(19) Europäisches Patentamt
European Patent Office
Office européen des brevets

(11) Veröffentlichungsnummer: **0 234 173 B1**

# (12) EUROPÄISCHE PATENTSCHRIFT

(45) Veröffentlichungstag der Patentschrift: **16.06.93**

(51) Int. Cl.5: **C07D 471/04**, A61K 31/435, //(C07D471/04,221:00,209:00)

(21) Anmeldenummer: **86730188.9**

(22) Anmeldetag: **11.11.86**

Die Akte enthält technische Angaben, die nach dem Eingang der Anmeldung eingereicht wurden und die nicht in dieser Patentschrift enthalten sind.

(54) **Phenoxy-substituierte beta-Carbolinderivate, ihre Herstellung und ihre Verwendung als Arzneimittel.**

(30) Priorität: **13.11.85 DE 3540654**

(43) Veröffentlichungstag der Anmeldung: **02.09.87 Patentblatt 87/36**

(45) Bekanntmachung des Hinweises auf die Patenterteilung: **16.06.93 Patentblatt 93/24**

(84) Benannte Vertragsstaaten: **AT BE CH DE ES FR GB GR IT LI LU NL SE**

(56) Entgegenhaltungen: **EP-A- 0 130 140**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Schmiechen, Ralph, Dr.**
**Bayernring 27**
**W-1000 Berlin 42(DE)**
Erfinder: **Seidelmann, Dieter, Dr.**
**Stierstrasse 14**
**W-1000 Berlin 41(DE)**
Erfinder: **Huth, Andreas, Dr.**
**Ermanstrasse 19**
**W-1000 Berlin 41(DE)**
Erfinder: **Schneider, Herbert Hans, Dr.**
**Duisburger Strasse 20**
**W-1000 Berlin 15(DE)**
Erfinder: **Stephens, David Norman, Dr.**
**Hildegardstrasse 16a**
**W-1000 Berlin 31(DE)**
Erfinder: **Engelstoft, Mogens, Dr.**
**Mosegard Park 121**
**DK-3500 Vaerlose(DK)**
Erfinder: **Hansen, John Bondo, Dr.**
**Harretoften 10**
**DK-2800 Lyngby(DK)**
Erfinder: **Petersen, Erling, Dr.**
**Platanhaven 109**
**DK-2600 Glostrup(DK)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue Phenoxy-substituierte β-Carbolin-Derivate, ihre Herstellung und ihre Verwendung als Arzneimittel.

In der EP-A-130140, die den 6-(4-Methoxy-phenoxy)-4-methoxymethyl-β-carbolin-3-carbonsäureethylester enthält und in der EP-A-54507, die den 5-Phenoxy-4-methyl-β-carbolin-3-carbonsäureethylester enthält, werden Verbindungen beschrieben, die die von β-Carbolinen bekannte Wirkung auf das Zentralnervensystem besitzen.

Die erfindungsgemäßen Verbindungen haben die allgemeine Formel I

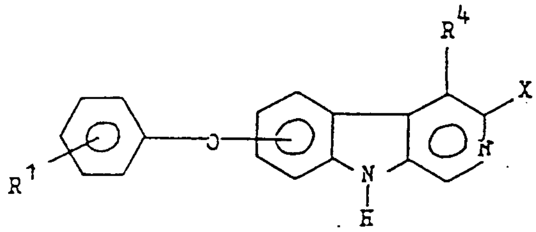 (I),

worin

X einen Oxadiazolylrest der Formel

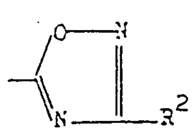 oder 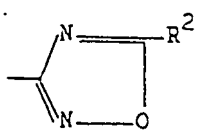

mit $R^2$ in der Bedeutung von H, $C_{1-6}$-Alkyl oder $C_{3-7}$-Cycloalkyl darstellt oder

X eine $COOR^3$-Gruppe mit $R^3$ in der Bedeutung von $C_{1-6}$-Alkyl darstellt oder $CONHC_{1-3}$-Alkyl ist und

$R^4$ Wasserstoff, $C_{1-6}$-Alkyl oder $C_{1-6}$-Alkoxyalkyl bedeutet und

$R^1$ Wasserstoff, Halogen, $C_{1-6}$-Alkyl $C_{1-6}$-Alkoxy, $C_{1-4}$-Alkanoyl, Phenyl, $C_{2-5}$-Alkylendioxy, Trifluormethyl, Cyano, Nitro, $C_{1-6}$-Alkoxycarbonyl, Azido, $SO_2R^6$, $SO_2NR^7R^8$ oder $NR^9R^{10}$ ist,

$R^6$ $C_{1-6}$-Alkyl,

$R^7$, $R^8$ $C_{1-6}$-Alkyl oder gemeinsam mit dem Stickstoffatom einen gesättigten 5- oder 6-gliedrigen Heterocyclus bedeuten, bei dem ein- oder zwei $CH_2$-Gruppen durch Sauerstoff, Schwefel oder Stickstoff ersetzt sein können, und

$R^9$, $R^{10}$ jeweils Wasserstoff, $C_{1-6}$-Alkyl, $C_{1-4}$-Alkanoyl oder gemeinsam mit dem Stickstoffatom einen gesättigten 5- oder 6-gliedrigen Heterocyclus bedeuten, bei dem ein- oder zwei $CH_2$-Gruppen durch Sauerstoff, Schwefel oder Stickstoff ersetzt sein können, und

$R^1$ gleich oder verschieden ein- bis dreifach stehen kann,

wobei X nicht $COOC_2H_5$ ist, wenn

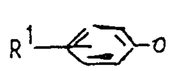 – 5-Phenoxy

und $R^4$ Methyl ist oder wenn

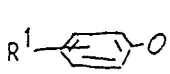 – 6-(4-Methoxyphenoxy)

und $R^4$ Methoxymethyl ist.

Die neuen β-Carbolinderivate der allgemeinen Formel I können im A-Ring in Position 5 - 8 ein- oder zweifach substituiert sein, wobei die Substitution in 5- oder 6-Stellung bevorzugt ist.

Der Substituent $R^1$ kann 1 oder mehrfach gleich oder verschieden am Arylrest auftreten, bevorzugt 1-3fach.

Unter niederem Alkyl sind sowohl gerad- als auch verzweigtkettige Reste mit $C_1$-$C_6$ Kohlenstoffatomen zu verstehen. Beispielsweise seien die bevorzugten $C_{1-4}$-Alkylreste genannt wie Methyl, Ethyl, n-Propyl, iso-Propyl, n-Butyl, iso-Butyl und tert.-Butyl und sek. Butyl.

Der Cycloalkylrest $R^2$ kann 3 - 7 Kohlenstoffatome enthalten, wobei als bevorzugt Reste mit 3 - 5 Kohlenstoffatomen genannt seien wie beispielsweise Cyclopropyl, Methylcyclopropyl, Cyclobutyl, Cyclopentyl u.a..

Der Acylrest leitet sich bevorzugt von aliphatischen Carbonsäuren mit bis zu 4 Kohlenstoffatomen ab wie beispielsweise Ameisensäure, Essigsäure, Propionsäure, Buttersäure u.a.

Bilden $R^7R^8$ und $R^9R^{10}$ gemeinsam mit dem Stickstoffatom einen Heterocyclus, so ist dieser 5 - 6 gliedrig und gesättigt, wobei eine oder 2 $CH_2$-Gruppen durch Sauerstoff, Schwefel oder Stickstoff ersetzt sein können.

Beispielsweise seien die folgenden Reste genannt: Imidazolidinyl, Pyrazolidinyl, Piperidinyl, Pyrrolidinyl, Piperazinyl, Morpholinyl, Thiomorpholinyl, Isothiazolidinyl u.a..

Unter Halogen sind Fluor, Chlor, Brom und Jod zu verstehen.

Die erfindungsgemäßen Verbindungen zeigen überraschenderweise im Vergleich zu den vorbekannten $\beta$-Carbolinen im pharmakologischen Test überlegene psychotrope Eigenschaften wie aus der Tabelle am Beispiel einiger erfindungsgemäßer Verbindungen zu ersehen ist.

Die erfindungsgemäßen Verbindungen zeigen insbesondere anxiolytische und antikonvulsive Wirksamkeit. Zur Untersuchung der antikonvulsiven Wirkung wurde die Aufhebung der mit Pentylentetrazol (Pentazol) induzierten Krämpfe untersucht. Pentazol wird in einer Menge von 150 mg/kg als Salzsäure-Lösung (pH 2-3) Subcutan 15-30 Minuten nach der intraperitonealen Applikation der Testsubstanz gegeben. Diese Menge induziert klonische und tonische Krämpfe, die bei unbehandelten Tieren zum Tode führen. Die Zahl der Mäuse, die Krämpfe zeigen und die Zahl derer, die 30 Minuten nach Pentazol gestorben sind, wird registriert (PTZ Krämpfe Antagonismus).

Die in der Tabelle angegebenen $ED_{50}$-Werte wurden nach der Methode von Litchfield und Wilcoxon (J. Pharmacol. exp. Ther. 96 (1949) 99-103) als die Menge der antagonistisch wirkenden Substanz bestimmt, die 50 % der Tiere vor Krämpfen und Tod schützt.

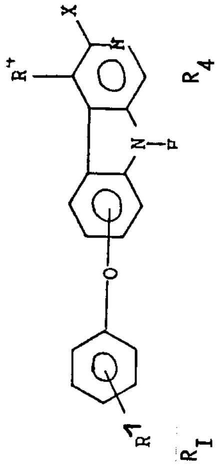

| $R_I$ | $R_4$ | X | $IC_{50}$ (ng/ml) in vitro | $ED_{50}$ (mg/kg) in vivo | PTZ ($ED_{50}$) mg/kg in vivo |
|---|---|---|---|---|---|
| 5 - Phenoxy | $CH_3$ | $COOC_2H_5$ | 2.0 | 2.8 | >30 |
| 5-(2-Cl-Phenoxy) | $CH_2OCH_3$ | 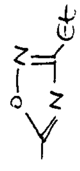 | 3.I | II | 0.8 |
| 5-(2-Cl-Phenoxy) | $CH_2OCH_3$ | $-COOCH(CH_3)_2$ | 2.6 | I9 | 4 |
| 5-(2-4-dichlorphenoxy) | $CH_2OCH_3$ | $-COOC_2H_5$ | 2.9 | 3.2 | 4 |
| 6-(2-nitrophenoxy) | $CH_2OCH_3$ | $-COOCH(CH_3)_2$ | 0.33 | I.8 | I3 |
| 6-(2-cyano- 3-Cl-Phenoxy) | $CH_2OCH_3$ | $-COOCH(CH_3)_2$ | 0.67 | 3.0 | I |
| 6-(2-cyanophenoxy) | $CH_2OCH_3$ | $-COOCH(CH_3)_2$ | 0.29 | 3.8 | 4 |
| 6-(4-acetylphenoxy) | $CH_2OCH_3$ | 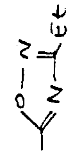 | 0.45 | 7.7 | 0.3 |
| 5-Phenoxy | $CH_2OCH_3$ | -COOEt | 0.3 | 0.4 | I8 |
| 5-Phenoxy | $CH_2OCH_3$ | 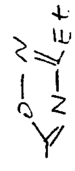 | 0.6 | 0.3 | 0.I |
| 5-Phenoxy | $CH_3$ | 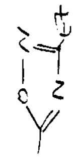 | 4.3 | 3.9 | 2 |
| 6-Phenoxy | $CH_3$ | 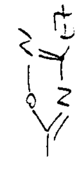 | 3.2 | 2.6 | 2.4 |

Es ist bekannt, daß bestimmte Stellen im zentralen Nervensystem von Vertebraten eine hohe spezifische Affinität für die Bindung von 1.4 und 1.5-Benzodiazepinen aufweisen (Squires, R.F. und Braestrup, C., Nature (London) 266 (1977), 734). diese Stellen werden Benzodiazepin-Rezeptoren genannt.

Die pharmakologischen Eigenschaften der erfindungsgemäßen Verbindungen wurden durch Untersuchung des Verdrängungsvermögens von radioaktiv-markiertem Flunitrazepam von Benzodiazepin-Rezeptoren bestimmt.

Die Verdrängungsaktivität der erfindugnsgemäßen Verbindugnen wird als $IC_{50}$ - und $ED_{50}$-Wert angegeben. Der $IC_{50}$-Wert gibt die Konzentration an, die eine 50 %ige Verdrängung der spezifischen Bindung von $H^3$-Flunitrazepam (1,0 nM, 0 °C) in Proben mit einem Gesamtvolumen von 0,55 ml einer Suspension von Hirnmembranen, z.B. von Ratten bewirkt.

Der Verdrängungstest wird wie folgt ausgeführt:
0,5 ml einer Suspension von unbehandeltem Rattenvorderhirn in 25 mM $KH_2PO_4$, pH = 7,1 (5-10 mg Gewebe/Probe) wird für 40 bis 60 Minuten bei 0 °C zusammen mit $^3$H-Diazepam (spezifische Aktivität 14,4 Ci/mmol, 1,9 nM) oder $^3$H-Flunitrazepam (spezifische Aktivität 87 Ci/mmol, 1,0 nM) inkubiert. Nach Inkubation wird die Suspension durch eine Glasfritte filtriert, der Rückstand zweimal mit kalter Pufferlösung gewaschen und die Radioaktivität im Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte kann dann der $IC_{50}$-Wert berechnet werden.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt:
Gruppen von Mäusen wird die Versuchssubstanz bei unterschiedlichen Dosen und normalerweise intraperitoneal injiziert. Nach 15 Minuten wird den Mäusen das $^3$H-Flunitrazepam intravenös verabreicht. Nach weiteren 20 Minuten werden die Mäuse getötet, ihr Vorderhirn entfernt und die an die Hirnmembranen spezifisch gebundene Radioaktivität durch Scintillationszählung gemessen. Der $ED_{50}$-Wert wird aus den Dosis/Wirkungs-Kurven bestimmt.

Die neuen Verbindungen der allgemeinen Formel I besitzen wertvolle pharmakologische Eigenschaften. Insbesondere wirken sie auf das Zentralnervensystem und sind somit als Psychopharmaka in der Humanmedizin geeignet, wobei sie insbesondere zur Behandlung von Angst begleitet von Depressionen, Epilepsie, Schlafstörungen, Spastizitäten und Muskelrelaxationen während der Anästhesie verwendet werden, Auch amnestische bzw. gedächtnisfördernde Eigenschaften werden bei erfindungsgemäßen Verbindungen gefunden.

Die erfindungsgemäßen Verbindungen können zur Formulierung von pharmazeutischen Präparationen, zum Beispiel für die orale und parenterale Anwendung am Menschen, nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindungsgemäßen Verbindungen werden in einer Dosis von 0,1 bis 300 mg/Tag, vorzugsweise 1-30 mg/Tag, angewendet.

Die Herstellung der erfindungsgemäßen Verbindungen der allgemeinen Formel I erfolgt nach an sich bekannten Methoden.
Beispielsweise erfolgt die Herstellung der Verbindung der allgemeinen Formel I, dadurch, daß man a) eine Verbindung der allgemeinen Formel II

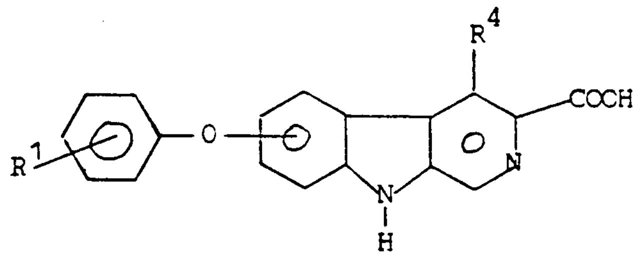

(II)

worin $R^1$ und $R^4$ die oben genannte Bedeutung haben mit einer Verbindung der Formel

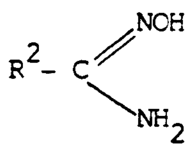

,

worin $R^2$ die oben genannte Bedeutung hat, umsetzt zu einer Verbindung der allgemeinen Formel I, in der X für den Rest

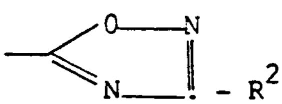

.

mit $R^2$ in der oben genannten Bedeutung steht,
b) eine Verbindung der allgemeinen Formel III

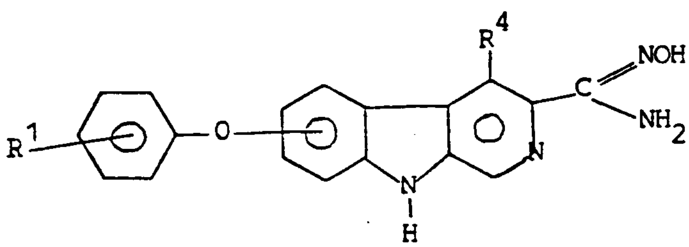

(III),

worin
$R^1$ und $R^4$ die oben genannte Bedeutung haben,
mit einem Carbonsäureanhydrid $(R^2CO)_20$, worin $R^2$ die oben genannte Bedeutung hat, umsetzt zu einer Verbindung der allgemeinen Formel I, in der X für den Rest

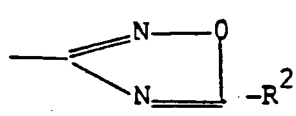

mit $R^2$ in der oben genannten Bedeutung steht,
c) eine Verbindung der allgemeinen Formel IV

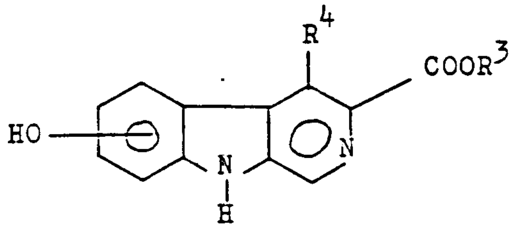

(IV),

worin $R^3$ und $R^4$ die oben genannte Bedeutung haben, mit einer Verbindung der Formel

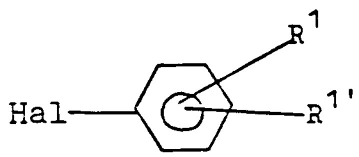

wobei $R^1$ die oben angegebene Bedeutung hat und $R^{1'}$ einen elektronenziehenden Substituenten darstellt, umsetzt und gegebenenfalls anschließend

α) eine Nitrogruppe zur Aminogruppe reduziert und gewünschtenfalls die so erhaltene Aminogruppe desaminiert oder gegen Halogen oder Azid austauscht oder

β) falls $R^1$ Halogen ist, katalytisch dehalogeniert oder

γ) eine Estergruppe umestert oder verseift und gewünschtenfalls die so erhaltene Carbonsäure amidiert.

Hal bedeutet Halogen, vorzugsweise Fluor und Chlor. Zur Einführung des 1,2,4-Oxadiazol-5-yl-Restes wird die β-Carbolincarbonsäure der allgemeinen Formel II mit einem Amidoxim der Formel

$R^2$-C( = NOH)$NH_2$,

in einem inerten Lösungsmittel, das über 100 °C siedet und gegenüber dem Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure zum Beispiel in das gemischte Anhydrid, in den aktivierten Ester oder in das Chlorid überführt werden.

Gut bewährt hat sich auch eine Aktivierung zum Imidazolid mit Imidazol/Thionylchlorid oder auch Carbonyldiimidazol in einem aprotischen Lösungsmittel wie Dioxan, Tetrahydrofuran, Dimethylformamid oder N-Methylpyrrolidon bei Temperaturen zwischen 0 und 50 °C, vorzugsweise Raumtemperatur.

Zur Einführung des 1,2,4-Oxadiazol-3-yl-Restes setzt man beispielsweise das 3-Carbonsäurenitril mit Hydroxylamin zu der Verbindung der allgemeinen Formel III um. Das so erhaltene β-Carbolin-3-carboxamidoxim wird bei Raumtemperatur mit dem Säureanhydrid $(R^2CO)_2O$ versetzt und anschließend bis zur Siedetemperatur erwärmt. Die Reaktion ist nach ca. 7 Stunden beendet und wird nach dem üblichen Verfahren aufgearbeitet.

Die Einführung des Phenoxyrestes erfolgt bevorzugt durch Umsetzung der Verbindung der allgemeinen Formel IV mit einem Fluorbenzolderivat, das zweckmäßigerweise einen weiteren elektronenziehenden Substituenten trägt.

Als elektronenziehende Substituenten $R^{1'}$ seien beispielsweise die folgenden für $R^1$ aufgeführten Reste genannt: Nitro. nieder Alkoxycarbonyl. nieder Alkylsulfonyl, Trifluormethyl, Cyano usw..

Die Umsetzung mit dem substituierten Halogenbenzolderivat wird im basischen Milieu in dipolaren aprotischen Lösungsmitteln bei Temperaturen bis zum Siedepunkt des Lösungsmittels vorgenommen.

Als Lösungsmittel sind beispielsweise Dimethylformamid, Dimethylsulfoxid, Dimethylacetamid, n-Methylpyrrolidinon, Hexamethylphosphorsäuretriamid u.a. geeignet.

Als Basen kommen Alkaliverbindungen wie beispielsweise Natrium- oder Kaliumhydroxid, Natrium- oder Kaliumcarbonat u.a. in Frage, gegebenenfalls auch in Gegenwart von Phasentransferkatalysatoren wie beispielsweise Kronenäther wie 18-Krone-6, Dicyclohexyl-18-Krone-6, Dibenzo-18-Krone-6 oder Aliquat 336.

Zweckmäßigerweise wird unter Inertgasatmosphäre, beispielsweise unter Stickstoff oder Argon, gearbeitet.

Die Reduktion der Nitrogruppe zur Aminogruppe erfolgt beispielsweise katalytisch in polaren Lösungsmitteln bei Raumtemperatur.

Vorzugsweise wird als Katalysator Palladium auf einem Träger wie Kohle oder Platin in feinverteilter Form verwendet; bei Verbindungen mit Halogen verwendet man als Katalysator vorzugsweise Raney-Nickel.

Alle inerten Lösungsmittel sind für die Reduktion geeignet wie beispielsweise Alkohole oder Aether wie Methanol, Ethanol, Diethylether, Tetrahydrofuran oder deren Mischungen u.a..

Die Hydrierung kann unter Normaldruck oder $H_2$-Druck vorgenommen werden.

Die Desaminierung erfolgt beispielsweise nach dem literaturbekannten Sandmeyer-Verfahren. Hierbei wird die mit einem Nitrit intermediär erzeugte Diazoniumverbindung in Gegenwart von Kupfer-I-oxid und hypophosphoriger Säure reduktiv bei erhöhter Temperatur verkocht.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure Salzsäure oder Bromwasserstoffsäure umsetzt oder mit Kaliumiodid umsetzt.

Die Einführung von Fluor gelingt beispielsweise durch Balz Schniemann-Reaktion des Diazoniumtetrafluorborates.

Die Einführung der Azidogruppe erfolgt über Sandmeyer-Reaktion des Diazoniumsalzes mit Alkaliazid zum Beispiel.

Die katalytische Dehalogenierung wird beispielsweise mit Palladium auf Kohle (10 %) unter Zusatz von organischen Basen wie beispielsweise Triethylamin,
u.a. in Alkoholen durchgeführt
Um Umesterungen zu vermeiden, nimmt man zweckmäßigerweise den Alkohol der Esterkomponente: als Lösungsmittel.

Wird eine Umesterung gewünscht, so kann man zum Beispiel mit dem entsprechenden Alkohol oder Alkalialkoholat umsetzen, gegebenenfalls kann man Titantetra-isopropylat als Katalysator im wasserfreien Alkohol zufügen. Üblicherweise wird die Umesterung bei Temperaturen von 60 - 120 °C durchgeführt und ist nach ca. 2 - 6 Stunden beendet.

Die Einführung der tert.-Butylestergruppe erfolgt beispielsweise durch Umsetzung der Carbonsäure mit tert.-Butoxy-bis-dimethylaminomethan. Im allgemeinen wird die Reaktion unter Inertgasatmosphäre wie Argon oder Stickstoff und unter Feuchtigkeitsausschluß bei erhöhter Temperatur durchgeführt.

So kann die Verseifung der Estergruppe sauer oder alkalisch erfolgen; vorzugsweise wird alkalisch verseift, indem Ester mit verdünnter wässriger Alkalilauge wie Kalium- oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Carbonsäureamide werden beispielsweise erhalten durch Umsetzung mit Aminen aus den entsprechenden Imidazoliden, die intermediär hergestellt werden aus den Carbonsäuren und Carbonyl- oder Thionyldiimidazol. Die Reaktion wird bei Raumtemperatur in dipolaren aprotischen Lösungsmitteln durchgeführt wie beispielsweise Dimethylformamid, Dimethylacetamid u.a..

Die Herstellung der Ausgangsverbindungen ist bekannt oder erfolgt nach bekannten Verfahren, wie beispielsweise in der EP-A-130140 beschrieben.
So können die Ester über Aktivierung der entsprechenden Säure und anschließende Umsetzung mit dem gewünschten Alkohol hergestellt werden.

Die nachfolgenden Beispiele sollen das erfindungsgemäße Verfahren erläutern.

Beispiel 1

5-(4-Chlorphenoxy)-3-(3-ethyl-1,2,4-oxadiazol-5-yl)-4-methoxymethyl-β-carbolin

5,74 g 5-(4-Chlorphenoxy)-4-methoxymethyl-β-carbolin-3-carbonsäure werden in 150 ml absolutem Dimethyformamid gelöst, mit 2,91 g Carbonyldiimidazol versetzt und 3 Stunden bei Raumtemperatur gerührt. Zu dieser Lösung gibt man 3,96 g Propionamidoxim, rührt 8 Stunden, gibt nochmals 1 g Propionamidoxim zu und rührt ebenfalls 8 Stunden. Nach Eindampfen am Ölpumpenvakuum wird in Toluol aufgenommen und 8 Stunden am Rückfluß gekocht. Nach Eindampfen wird zweimal über Kieselgel zunächst mit Methylenchlorid/Ethanol = 10 : 1 und dann mit Hexan : Aceton = 1 : 1 als Elutionsmittel chromatographiert. Nach Umkristallisation aus Essigester/Hexan und Trocknen über Phosphorpentoxid bei 80 °C am Vakuum erhält man 2,2 g 5-(4-Chlorphenoxy-3-(3-ethyl-1,2,4-oxidiazol-5-yl)-4-methoxymethyl-β-carbolin vom Schmelzpunkt 170 °C.

In analoger Weise werden hergestellt:

5-Phenoxy-4-methyl-3-(3-ethyl-1,2,4-oxadiazol-5-yl)-β-carbolin
Schmelzpunkt 245 - 248 °C
5-(4-Nitrophenoxy)-3-(3-ethyl-1,2,4-oxadiazol-5-yl)-β-carbolin
Schmelzpunkt 290 °C
5-(4-Chlorphenoxy)-4-methyl-3-(3-ethyl-1,2,4-oxadiazol-5-yl)-β-carbolin
Schmelzpunkt 193 - 194 °C
6(4-Acetylphenoxy)-4-methoxymethyl-3-(3-ethyl-1,2,4-oxadiazol-5 yl)-β-carbolin
Schmelzpunkt 213 - 216 °C
5-(4-Nitrophenoxy)-4-methyl-3-(3-ethyl-1,2,4-oxadiazol-5-yl)-β-carbolin
Schmelzpunkt 287 °C
5-(4-Nitrophenoxy)-4-methoxymethyl-3-(3-ethyl-1,2,4-oxadiozol-5-yl)-β-carbolin
Schmelzpunkt 125 - 180 °C
5-Phenoxy-4-methoxymethyl-3-(3-ethyl-1,2,4-oxadiazol-5-yl)-β-carbolin
Schmelzpunkt 168 - 171 °C
6-Phenoxy-3-(3-ethyl-1,2,4-oxadiazol-5-yl)-β-carbolin
Schmelzpunkt 205 - 208 °C
6-Phenoxy-4-methyl-3-(3-ethyl-1,2,4-oxadiazol-5-yl)-β-carbolin
Schmelzpunkt 247 - 250 °C
6-(4-Nitrophenoxy)-3-(3-ethyl-1,2,4-oxadiazol-5-yl)-β-carbolin
Schmelzpunkt 288 - 294 °C
6-(4-Aminophenoxy)-3-(3-ethyl-1,2,4-oxadiazol-5-yl)-β-carbolin
Schmelzpunkt 207 - 210 °C
6-(4-Chlorphenoxy)-4-methyl-3-(3-ethyl-1,2,4-oxadiazol-5-yl)-β-carbolin
Schmelzpunkt 245 - 250 °C
6-(4-Nitrophenoxy)-4-methyl-3-(3-ethyl-1,2,4-oxadiazol-5-yl)-β-carbolin
Schmelzpunkt 250 - 258 °C
6-(4-Aminophenoxy)-4-methyl-3-(3-ethyl-1,2,4-oxadiazol-5-yl)-β-carbolin
Schmelzpunkt 245 - 255 °C
6-(4-Chlorphenoxy)-4-methoxymethyl-3-(3-ethyl-1,2,4-oxadiazol-5-yl)-β-carbolin
Schmelzpunkt 178 - 192 °C
6-(4-Chlorphenoxy)-4-methoxymethyl-3-(3-cyclopropyl-1,2,4-oxadiazol-5-yl-)-β-carbolin
Schmelzpunkt 192 - 193 °C
6-(4-Bromphenoxy)-4-methoxymethyl-3-(3-ethyl-1,2,4-oxadiazol-5-yl)-β-carbolin
Schmelzpunkt > 280 °C
6-Phenoxy-4-methoxymethyl-3-(3-ethyl-1,2,4-oxadiazol-5-yl)-β-carbolin
Schmelzpunkt 164 °C
6-(4-Nitrophenoxy)-4-methoxymethyl-3-(3-ethyl-1,2,4-oxadiazol-5-yl)-β-carbolin
Schmelzpunkt 223 - 225 °C
6-(4-Nitrophenoxy)-4-methoxymethyl-3-(3-cyclopropyl-1,2,4-oxadiazol-5-yl)-β-carbolin
Schmelzpunkt 226 °C
6-(2-Nitrophenoxy)-4-methoxymethyl-3-(3-ethyl-1,2,4-oxadiazol-5-yl)-β-carbolin
Schmelzpunkt 206 - 211 °C
6-(2-Nitrophenoxy)-4-methoxymethyl-3-(3-cyclopropyl-1,2,4-oxadiazol-5yl)-β-carbolin
Schmelzpunkt 154 - 155 °C
6-(4-Aminophenoxy)-4-methoxymethyl-3-(3-ethyl-1,2,4-oxadiazol-5-yl)-β-carbolin
Schmelzpunkt 249 - 255 °C
6-(2-Methyl-4-nitrophenoxy)-4-methoxymethyl-3-(3-ethyl-1,2,4-oxadiazol-5-yl)-β-carbolin
Schmelzpunkt 208 - 209 °C
6-(4-Morpholinosulfamoylphenoxy)-4-methoxymethyl-3-(3-ethyl-1,2,4-oxadiazol-5-yl)-β-carbolin
Schmelzpunkt 113 °C
6-(4-Morpholinosulfamoylphenoxy)-4-methoxymethyl-3-(3-cyclopropyl-1,2,4-oxadiazol-5-yl)-β-carbolin
Schmelzpunkt 139 - 140 °C
6-(4-Diethylsulfamoylphenoxy)-4-methoxymethyl-3-(3-ethyl-1,2,4-oxadiazol-5-yl)-β-carbolin
Schmelzpunkt 184 - 189 °C
6-(4-Methylsulfonylphenoxy)-4-methoxymethyl-3-(3-ethyl-1,2,4-oxadiazol-5-yl)-β-carbolin
Schmelzpunkt 150 °C
6-(4-Ethoxycarbonylphenoxy)-4-methoxymethyl-3-(3-ethyl-1,2,4-oxadiazol-5-yl)-β-carbolin

Schmelzpunkt 185 - 191 °C
6-(2-Chlorphenoxy)-4-methoxymethyl-3-(3-ethyl-1,2,4-oxadiazol-5-yl)-β-carbolin
Schmelzpunkt 158 - 161 °C
6-(4-Cyanophenoxy)-4-methoxymethyl-3-(3-ethyl-1,2,4-oxadiazol-5-yl)-β-carbolin
Schmelzpunkt 224 - 225 °C
6-(2-Chlor-4-nitrophenoxy)-4-methoxymethyl-3-(3-ethyl-1,2,4-oxadiazol-5-yl)-β-carbolin
Schmelzpunkt 200 - 213 °C
6-(2-Chlor-4-aminophenoxy)-4-methoxymethyl-3-(3-ethyl-1,2,4-oxadiazol-5-yl)-β-carbolin
Schmelzpunkt 235 - 247 °C
6-(2,4-Dichlorphenoxy)-4-methoxymethyl-3-(3-ethyl-1,2,4-oxadiazol-5-yl)-β-carbolin
Schmelzpunkt 160 - 174 °C
6(4-Fluorphenoxy)-4-methoxymethyl-3-(3-ethyl-1,2,4-oxadiazol-5 yl)β-carbolin
Schmelzpunkt 240 - 242 °C
5(3-Chlorphenoxy)-4-methoxymethyl-3-(3-ethyl-1,2,4-oxadiazol-5 yl)-β-carbolin
Schmelzpunkt 170 - 172 °C

Beispiel 2

4-Methoxymethyl-5-phenoxy-3-(3-(5-ethyl-1,2,4-oxadiazol)-yl-β-carbolin

0,7 mMol 4-Methoxymethyl-5-phenoxy-β-carbolin-3-carboxamidoxim und 1 ml Propionsäureanhydrid werden für 2 Stunden bei 20 °C und danach 5 Stunden bei 120 °C gerührt. Nach dem Einengen werden 10 ml Tetrahydrofuran hinzugefügt, die Reaktionsmischung über Nacht stehengelassen, danach im Vakuum konzentriert und das Reaktionsprodukt mit 30 ml Methylenchlorid als ölige Substanz extrahiert.

Das Ausgangsmaterial wird wie folgt hergestellt:

a) 5-Phenoxy-4-methoxymethyl-β-carbolin-3-carboxamid

Zur Lösung von 30 mMol Thionyldiimidazol in 150 ml Tetrahydrofuran werden 2,7 g 5-Phenoxy-4-methoxymethyl-β-carbolin-3-carbonsäure gegeben. Die Reaktionsmischung wird 5 Stunden gerührt und filtriert. Das Filtrat wird mit 12 ml 25 prozentigen $NH_3$ in Wasser versetzt, über Nacht gerührt und im Vakuum auf 50 ml eingeengt. Nach Zugabe von 100 ml Wasser werden 2 g des gewünschten Produktes als gelbe Kristalle erhalten.

b) 5-Phenoxy-3-cyano-4-methoxymethyl-β-carbolin

Zu einer gerührten Lösung von 1,8 g (15 mMol) Triphenylphosphin in 50 ml Methylenchlorid werden bei 0 °C 1,1 g $Br_2$ in 10 ml Methylenchlorid getropft. Danach werden 2 g 5-Phenoxy-4-methoxymethyl-β-carbolin-3-carboxamid und 1,9 ml Triäthylamin hinzugesetzt. Die Reaktionsmischung wird bei 0 °C für 1 Stunde gerührt und danach mit 25 ml Methylenchlorid und 25 ml Wasser 5 Minuten stark gerührt. Nach Entfernung der wässrigen Phase erhält man aus der organischen Phase durch Einengen 0,8 g gewünschtes Produkt.

c) 4-Methoxymethyl-5-phenoxy-β-carbolin-3-carboxamidoxim

Eine Mischung von 329 mg (0,001 Mol) 3-Cyano-4-methoxymethyl-5-phenoxy-β-carbolin, 100 mg Hydroxylaminhydrochlorid, 20 ml Äthanol (99 %) und 0,52 ml einer 20 prozentigen wässrigen Kaliumcarbonatlösung wird 22 Stunden unter Rückfluß gehalten. Die Reaktionsmischung wird filtriert und das Filtrat eingeengt. Der Rückstand wird mit 10 ml Wasser versetzt, der kristalline Feststoff abfiltriert und mit Wasser gewaschen.

Beispiel 3

5-(4-Nitrophenoxy)-4-methoxymethyl-β-carbolin-3-carbonsäureethylester

6 g 5-Hydroxy-4-methoxymethyl-β-carbolin-3-carbonsäureethylester werden unter Stickstoff in 200 ml Dimethylformamid mit 5,5 g wasserfreien Kaliumcarbonat versetzt und 1 Stunde bei Raumtemperatur gerührt.

Nach Versetzen mit 2,8 g 4-Fluornitrobenzol wird 2 Stunden auf 100 °C Badtemperatur erwärmt. Nach nochmaliger Zugabe von 1,4 g 4-Fluornitrobenzol wird nochmals 45 Minuten auf 100 °C erwärmt. Nach Abkühlen wurde auf Eis gegossen und abgesaugt. Der Filterkuchen wird über Kieselgel mit Aceton : Hexan = 1 : 1 als Laufmittel chromatographiert. Man erhält 5,7 g (70 % d.Th.) 5-(4-Nitrophenoxy)-4-methoxymethyl-β-carbolin-3-carbonsäureethylester vom Schmelzpunkt 231 - 232 °C.

In analoger Weise werden hergestellt:

5-(2-Nitrophenoxy)-4-methyl-β-carbolin-3-carbonsäureethylester
Schmelzpunkt 241 - 242 °C
5-(2-Nitrophenoxy)-4-ethyl-β-carbolin-3-carbonsäureethylester
6-3(4-Cyanophenoxy)-4-methoxymethyl-β-carbolin-3-carbonsäureethylester
Schmelzpunkt 226 - 227 °C
6-(2-Nitrophenoxy)-4-methoxymethyl-β-carbolin-3-carbonsäure-isopropylester
Schmelzpunkt 147 - 150 °C
6-(2-Formylphenoxy)-4-methoxymethyl-β-carbolin-3-carbonsäureisopropylester
Schmelzpunkt 188 - 192 °C
6-(2-Cyanophenoxy)-4-methoxymethyl-β-carbolin-3-carbonsäureisopropylester
Schmelzpunkt 170 °C
6-(2-Cyano-3-chlorphenoxy)-4-methoxymethyl-β-carbolin-3-carbonsäureisopropylester
Schmelzpunkt 117 - 125 °C
6-(2-Acetylphenoxy)-4-methoxymethyl-β-carbolin-3-carbonsäureisopropylester
Schmelzpunkt 112 - 117 °C
6-(2-Cyano-4-fluorophenoxy)-4-methoxymethyl-β-carbolin-3-carbonsäureisopropylester
Schmelzpunkt 228 - 230 °C
6-(4-Acetylphenoxy)-4-methoxymethyl-β-carbolin-3-carbonsäureisopropylester
Schmelzpunkt 233 °C
5-(4-Nitrophenoxy)-4-methyl-β-carbolin-3-carbonsäureethylester
Schmelzpunkt 225 °C
5-(4-Nitrophenoxy)-4-ethyl-β-carbolin-3-carbonsäureethylester
Schmelzpunkt 217 - 218 °C
5-(4-Nitrophenoxy)-β-carbolin-3-carbonsäureethylester
Schmelzpunkt >242 °C
5-(4-Nitro-2-chlorphenoxy)-4-methoxymethyl-β-carbolin-3-carbonsäureethylester
5-(4-Nitro-3-methylphenoxy)-4-methoxymethyl-β-carbolin-3-carbonsäureethylester
Schmelzpunkt 212 - 213 °C
5-(4-Nitro-2-methylphenoxy)-4-methoxymethyl-β-carbolin-3-carbonsäureethylester
Schmelzpunkt 190 - 192 °C
5-(4-Ethoxycarbonylphenoxy)-4-methoxymethyl-β-carbolin-3-carbonsäureethylester
Schmelzpunkt 157 °C
6-(4-Nitrophenoxy)-β-carbolin-3-carbonsäuremethylester
6-(4-Nitrophenoxy)-β-carbolin-3-carbonsäureethylester
Schmelzpunkt >25o °C
6-(4-Nitrophenoxy)-4-methyl-β-carbolin-3-carbonsäureethylester
Schmelzpunkt 288 - 292 °C
6-(4-Nitrophenoxy)-4-methoxymethyl-β-carbolin-3-carbonsäureethylester
Schmelzpunkt 231 - 232 °C
6-(2-Cyano-3-chlorphenoxy)-4-methyl-β-carbolin-3-carbonsäureisopropylester
Schmelzpunkt 230 - 232 °C
6-(2-Cyano-6-fluorophenoxy)-4-methoxymethyl-β-carbolin-3-carbonsäureisopropylester
Schmelzpunkt 175 °C
6-(2-cyano-3-fluorophenoxy)-4-methoxymethyl-β-carbolin-3-carbonsäureisopropylester
Schmelzpunkt 208 °C
6-(2-Isopropoxycarbonylphenoxy)-4-methoxymethyl-β-carbolin-3-carbonsäureisopropylester
Schmelzpunkt 145 °C
6-(2-t-Butoxycarbonylphenoxy)-4-methoxymethyl-β-carbolin-3-carbonsäureisopropylester
Schmelzpunkt 136 °C
6-(4-Fluoro-2-nitrophenoxy)-4-methoxymethyl-β-carbolin-3-carbonsäureisopropylester
6-(4-Nitro-3-chlorophenoxy)-4-methoxymethyl-β-carbolin-3-carbonsäureisopropylester
6-(4-Nitro-3-methylphenoxy)-4-methoxymethyl-β-carbolin-3-carbonsäureisopropylester

6-(2-Nitro-3-chlorphenoxy)-4-methoxymethyl-β-carbolin-3-carbonsäureisopropylester
6-(2-Nitro-3,5-dichlorphenoxy)-4-methoxymethyl-β-carbolin-3-carbonsäureisopropylester
6-(2-Nitrophenoxy)-4-methoxymethyl-β-carbolin-3-carbonsäureethylester
Schmelzpunkt 153 - 155 °C
6-(4-Nitro-3-methoxyphenoxy)-4-methoxymethyl-β-carbolin-3-carbonsäureethylester
Schmelzpunkt 192 - 203 °C
6-(4-Nitro-2-methylphenoxy)-4-methoxymethyl-β-carbolin-3-carbonsäureethylester
Schmelzpunkt 184 - 185 °C
6-(4-Nitro-2-chlorphenoxy)-4-methoxymethyl-β-carbolin-3-carbonsäureethylester
Schmelzpunkt 195 °C
6-(4-Nitro-3-methyl-phenoxy)-4-methoxymethyl-β-carbolin-3-carbonsäureethylester
Schmelzpunkt 183 - 184 °C
6-(2-Nitro-4-trifluormethylphenoxy)-4-methoxymethyl-β-carbolin-3-carbonsäureethylester
Schmelzpunkt 90 °C
6-(4-Ethoxycarbonylphenoxy)-4-methoxymethyl-β-carbolin-3-carbonsäureethylester
Schmelzpunkt 181 °C
6-(4-Trifluormethylphenoxy)-4-methoxymethyl-β-carbolin-3-carbonsäureethylester
Schmelzpunkt 226 - 227 °C
6-(4-Methylsulfonylphenoxy)-4-methoxymethyl-β-carbolin-3-carbonsäureethylester
Schmelzpunkt 204 - 205 °C
5(4-Formylphenoxy)-4-methoxymethyl-β-carbolin-3-carbonsäureethylester
Schmelzpunkt 190 - 192 °C
5(2-Nitro-4-chlorphenoxy)-4-methoxymethyl-β-carbolin-3-carbonsäureethylester
Schmelzpunkt 160 - 162 °C
5(2-Nitro-5-chlorphenoxy)-4-methoxymethyl-β-carbolin-3-carbonsäureethylester
Schmelzpunkt 155 - 170 °C
6-(4-Nitro-2-methylphenoxy)-4-methoxymethyl-β-carbolin-3-carbonsäureisopropylester
6-(2-Nitrophenoxy)-4-ethyl-β-carbolin-3-carbonsäureethylester
6-(2-Nitro-4-methoxyphenoxy)-4-methoxymethyl-β-carbolin-3-carbonsäureisopropylester
6-(4-Nitro-3-methoxyphenoxy)-4-methoxymethyl-β-carbolin-3-carbonsäureisopropylester
6-(4-Nitro-3-cyanophenoxy)-4-methoxymethyl-β-carbolin-3-carbonsäureisopropylester
6-(2-Nitro-4-methylphenoxy)-4-methoxymethyl-β-carbolin-3-carbonsäureisopropylester
6-(2-Methoxy-4-Nitrophenoxy)-4-methoxymethyl-β-carbolin-3-carbonsäureisopropylester
6-(4-Morpholinosulfamoylphenoxy)-4-methoxymethyl-β-carbolin-3-carbonsäureethylester
Schmelzpunkt 100 °C (Zers.)
6-(4-Diethylsulfamoylphenoxy)-4-methoxymethyl-β-carbolin-3-carbonsäureethylester
Schmelzpunkt 179 °C
6-(2-Ethylsulfonylphenoxy)-4-methoxymethyl-β-carbolin-3-carbonsäureethylester
Schmelzpunkt 196 - 198 °C

## Beispiel 4

### 5-(4-Aminophenoxy)-4-methoxymethyl-β-carbolin-3-carbonsäureethylester

15 g 5-(4-Nitrophenoxy)-4-methoxymethyl-β-carbolin-3-carbonsäureethylester werden in 450 ml Methanol : Tetrahydrofuran = 1 : 1 mit 7,5 g Palladium auf Kohle (10 %) bei Raumtemperatur unter Wasserstoffnormaldruck hydriert. Nach Filtrieren und Einengen wird aus Ethanol umkristallisiert und man erhält 10,8 g (77 % d.Th.) 5-(4-Aminophenoxy)-4-methoxymethyl-β-carbolin-3-carbonsäureethylester vom Schmelzpunkt 222 - 224 °C.

In analoger Weise werden hergestellt:
5-(4-Aminophenoxy)-4-methyl-β-carbolin-3-carbonsäureethylester
Schmelzpunkt 170 - 172 °C
5-(4-Aminophenoxy)-4-ethyl-β-carbolin-3-carbonsäureethylester
Schmelzpunkt 235 °C
6-(4-Aminophenoxy)-4-methyl-β-carbolin-3-carbonsäureethylester
6-(4-Aminophenoxy)-4-methoxymethyl-β-carbolin-3-carbonsäureethylester
Schmelzpunkt 204 - 234 °C
6-(4-Amino-3-chlorophenoxy)-4-methoxymethyl-β-carbolin-3-carbonsäureisopropylester

6-(4-Amino-3-methylphenoxy)-4-methoxymethyl-β-carbolin-3-carbonsäureisopropylester
6-(2-Amino-3-chlorphenoxy)-4-methoxymethyl-β-carbolin-3-carbonsäureisopropylester
6-(2-Amino-3,5-dichlorphenoxy)-4-methoxymethyl-β-carbolin-3-carbonsäureisopropylester
5-(2-Amino-5-chlorophenoxy)-4-methoxymethyl-β-carbolin-3-carbonsäureethylester
6-(4-Amino-2-methylphenoxy)-4-methoxymethyl-β-carbolin-3-carbonsäureisopropylester
6-(4-Fluoro-2-aminophenoxy)-4-methoxymethyl-β-carbolin-3-carbonsäureisopropylester
6-(4-Amino-2-chlorphenoxy)-4-methoxymethyl-β-carbolin-3-carbonsäureisopropylester
6-(2-Amino-4-methoxyphenoxy)-4-methoxymethyl-β-carbolin-3-carbonsäureisopropylester
6-(4-Amino-3-methoxyphenoxy)-4-methoxymethyl-β-carbolin-3-carbonsäureisopropylester
6-(4-Amino-3-cyanophenoxy)-4-methoxymethyl-β-carbolin-3-carbonsäureisopropylester
6-(2-Amino-4-methylphenoxy)-4-methoxymethyl-β-carbolin-3-carbonsäureisopropylester
6-(2-Methoxy-4-aminophenoxy)-4-methoxymethyl-β-carbolin-3-carbonsäureisopropylester
5-(4-Aminophenoxy)-4-methoxymethyl-β-carbolin-3-carbonsäureisopropylester
Schmelzpunkt > 250 °C

In grundsätzlich analoger Weise, allerdings mit Raney Nickel als Katalysator und Tetrahydrofuran als Lösungsmittel, werden hergestellt:

5-(4-Amino-3-Chlorphenoxy)-4-methoxymethyl-β-carbolin-3-carbonsäureethylester
Schmelzpunkt 202 - 204 °C
5-(4-Amino-2-chlorphenoxy)-4-methoxymethyl-β-carbolin-3-carbonsäureethylester
Schmelzpunkt 204 °C
6-(4-Amino-2-chlorphenoxy)-4-methoxymethyl-β-carbolin-3-carbonsäureethylester
Schmelzpunkt 95 - 106 °C

Beispiel 5

5-Phenoxy-4-methoxymethyl-β-carbolin-3-carbonsäureethylester

978 mg 5-(4-Aminophenoxy)-4-methoxymethyl-β-carbolin-3-carbonsäureethylester werden in 2 ml Wasser und 10 ml einer 50 prozentigen Tetrafluorborsäure suspendiert. Nach Abkühlen auf 0 °C wird tropfenweise mit einer Lösung von 224 mg Natriumnitrit in 2 ml Wasser versetzt und bei 0 °C 1/2 Stunde gerührt. Anschliessend wird bei der gleichen Temperatur mit 4 ml einer 60 prozentigen hypophosphorigen Säure und 150 mg Kupfer(I)oxid versetzt, mit 10 ml Wasser verdünnt und darauf 1/2 Stunde auf dem Dampfbad erwärmt. Nach Einstellen des pH-Wertes auf 8 mit Soda und Zugabe von Ammoniak wird mit Essigester extrahiert. Die Essigesterphase wird eingedampft und der Rückstand über Kieselgel mit Aceton : Hexan = 1 : 1 als Elutionsmittel chromatographiert. Man erhält 540 mg (57 % d.Th.) 5-Phenoxy-4-methoxymethyl-β-carbolin-3-carbonsäureethylester vom Schmelzpunkt 174 - 176 °C.

In analoger Weise werden hergestellt:

5-Phenoxy-β-carbolin-3-carbonsäureethylester
Schmelzpunkt 246 °C
5-(3-Chlorphenoxy)-4-methoxymethyl-β-carbolin-3-carbonsäureethylester
Schmelzpunkt 194 - 197 °C
5-(2-Chlorphenoxy)-4-methoxymethyl-β-carbolin-3-carbonsäureethylester
Schmelzpunkt 175 - 177 °C
6-Phenoxy-β-carbolin-3-carbonsäureethylester
Schmelzpunkt 241 - 242 °C
6-Phenoxy-4-methyl-β-carbolin-3-carbonsäureethylester
6-Phenoxy-4-methoxymethyl-β-carbolin-3-carbonsäureethylester
Schmelzpunkt 172 - 174 °C
6-(3-Chlorphenoxy)-4-methoxymethyl-β-carbolin-3-carbonsäureisopropylester
Schmelzpunkt 164 °C
6-(3-methylphenoxy)-4-methoxymethyl-β-carbolin-3-carbonsäureisopropylester
Schmelzpunkt 170 - 174 °C
6-(3,5-Dichlorphenoxy)-4-methoxymethyl-β-carbolin-3-carbonsäureisopropylester
Schmelzpunkt 210 °C
6-(2-Methylphenoxy)-4-methoxymethyl-β-carbolin-3-carbonsäureisopropylester
Schmelzpunkt 165 - 170 °C
5-(2,5-Dichlorphenoxy)-4-methoxymethyl-β-carbolin-3-carbonsäureethylester
Schmelzpunkt 194 - 196 °C

6-(4-Methoxyphenoxy)-4-methoxymethyl-β-carbolin-3-carbonsäureisopropylester
6-(3-Cyanophenoxy)-4-methoxymethyl-β-carbolin-3-carbonsäureisopropylester
6-(3-Methoxyphenoxy)-4-methoxymethyl-β-carbolin-3-carbonsäureisopropylester
6-(4-Methylphenoxy)-4-methoxymethyl-β-carbolin-3-carbonsäureisopropylester
6-(2-Methoxyphenoxy)-4-methoxymethyl-β-carbolin-3-carbonsäureisopropylester

Beispiel 6

5-(4-Chlorphenoxy)-4-methoxymethyl-β-carbolin-3-carbonsäureethylester

195 mg 5-(4-Aminophenoxy)-4-methoxymethyl-β-carbolin-3-carbonsäureethylester werden in einer Mischung aus 2 ml Wasser und 2 ml konzentrierter Salzsäure suspendiert und nach Kühlung auf 0 °C tropfenweise mit einer Lösung aus 35 mg Natriumnitrit in 0,5 ml Wasser versetzt. Nach beendeter Zugabe wird 45 Minuten bei 0 °C gerührt, wobei eine hellgelbe Lösung entsteht. Dazu wird bei 0 °C eine Lösung getropft, die zuvor durch Zugabe von 69 mg Natriumsulfit in 0,5 ml Wasser zu 250 mg Kupfer)II)sulfat, $5H_2O$ und 87 mg Kochsalz in 1 ml Wasser, Absaugen des Niederschlages und Auflösen in 0,5 ml konzentrierter Salzsäure hergestellt wurde. Nach beendeter Zugabe ist eine gelbe Fällung eingetreten und das Gemisch wird dann bis zum Ende der Gasentwicklung auf dem Dampfbad erhitzt. Anschließend wird mit Wasser verdünnt, mit Ammoniaklösung alkalisch gestellt und mit Essigester extrahiert. Nach Eindampfen der organischen Phase wird über Kieselgel mit Methylenchlorid : Ethanol = 10 : 1 als Elutionsmittel chromatographiert. Man erhält 130 mg (55 % d.Th.) 5-(4-Chlorphenoxy)-4-methoxymethyl-β-carbolin-3-carbonsäureethylester vom Schmelzpunkt 207 °C.

In analoger Weise werden hergestellt:
5-(4-Chlorphenoxy)-4-methyl-β-carbolin-3-carbonsäureethylester
5-(2,4-Dichlorphenoxy)-4-methoxymethyl-β-carbolin-3-carbonsäureethylester
Schmelzpunkt 156 - 158 °C
6-(4-Chlorphenoxy)-4-methyl-β-carbolin-3-carbonsäureethylester Schmelzpunkt 176 - 188 °C
6-(4-Chlorphenoxy)-4-methoxymethyl-β-carbolin-3-carbonsäureethylester
Schmelzpunkt 178 °C
6-(2,4-Dichlorphenoxy)-4-methoxymethyl-β-carbolin-3-carbonsäureethylester
5-(4-Jodphenoxy)-4-methoxymethyl-β-carbolin-3-carbonsäureethylester
Schmelzpunkt 200 °C
6-(2-Bromphenoxy)-4-methoxymethyl-β-carbolin-3-carbonsäureisopropylester
Schmelzpunkt 152 - 160 °C
6-(4-Fluoro-2-chlorophenoxy)-4-methoxymethyl-β-carbolin-3-carbonsäureisopropylester
Schmelzpunkt 134 - 144 °C
6-(2,3-Dichlorphenoxy)-4-methoxymethyl-β-carbolin-3-carbonsäureisopropylester
Schmelzpunkt 100 - 101 °C
6-(4-Piperidinoazophenoxy)-4-methoxymethyl-β-carbolin-3-carbonsäureisopropylester
Schmelzpunkt 168 - 174 °C
6-(4-Bromphenoxy)-4-methoxymethyl-β-carbolin-3-carbonsäureethylester
Schmelzpunkt 169 - 175 °C
6-(4-Azidophenoxy)-4-methoxymethyl-β-carbolin-3-carbonsäureethylester
Schmelzpunkt 169 - 175 °C

Beispiel 7

5-Phenoxy-4-methoxymethyl-β-carbolin-3-carbonsäureisopropylester

540 mg 5-Phenoxy-4-methoxymethyl-β-carbolin-3-carbonsäureethylester werden in 30 ml Isopropnaol mit 0,2 ml Titan(IV)isopropylat 2 Stunden am Rückfluß gekocht. Nach Einengen wird mit 0,5 n Salzsäure versetzt und mit Essigester extrahiert. Die Essigesterphase wird getrocknet, filtriert und eingeengt und mit Diisopropylether digeriert. Man erhält 450 mg (82% d.Th.) 5-Phenoxy-4-methoxymethyl-β-carbolin-3-carbonsäureisopropylester vom Schmelzpunkt 207 - 209 °C.

In analoger Weise werden hergestellt:
5-Phenoxy-4-methyl-β-carbolin-3-carbonsäureisopropylester
5-(4-Chlorphenoxy)-4-methyl-β-carbolin-3-carbonsäureisopropylester
Schmelzpunkt 266 - 268 °C

5-(4-Chlorphenoxy)-4-methoxymethyl-β-carbolin-3-carbonsäureisopropylester
Schmelzpunkt 216 - 218 °C
5-(4-Nitrophenoxy)-4-methyl-β-carbolin-3-carbonsäureisopropylester
Schmelzpunkt 262 °C
5-(4-Nitrophenoxy)-4-methoxymethyl-β-carbolin-3-carbonsäureisopropylester
Schmelzpunkt 207 - 209 °C
6-Phenoxy-4-methoxymethyl-β-carbolin-3-carbonsäureisopropylester
Schmelzpunkt 181 °C
6-(4-Chlorphenoxy)-4-methoxymethyl-β-carbolin-3-carbonsäureisopropylester
Schmelzpunkt 178 - 181 °C
6-(4-Cyanophenoxy)-4-methoxymethyl-β-carbolin-3-carbonsäureisopropylester
Schmelzpunkt 226 - 227 °C
6-(2-Chlorphenoxy)-4-methoxymethyl-β-carbolin-3-carbonsäureisopropylester
Schmelzpunkt 103 - 109 °C
6-(4-Isopropoxycarbonylphenoxy)-4-methoxymethyl-β-carbolin-3-carbonsäureisopropylester
Schmelzpunkt 167 °C
6-(3-Chlor-4-nitrophenoxy)-4-methoxymethyl-β-carbolin-3-carbonsäureisopropylester
Schmelzpunkt 105 - 115 °C
6-(2,4-Dichlorphenoxy)-4-methoxymethyl-β-carbolin-3-carbonsäureisopropylester
Schmelzpunkt 75 - 78 °C
6-(4-Fluorphenoxy)-4-methoxymethyl-β-carbolin-3-carbonsäureisopropylester
Schmelzpunkt 104 - 116 °C
5(3-Chlorphenoxy)-4-methoxymethyl-β-carbolin-3-carbonsäureisopropylester
Schmelzpunkt 187 - 189 °C
6-(3,4-Dichlorphenoxy)-4-methoxymethyl-β-carbolin-3-carbonsäureisopropylester
Schmelzpunkt 66 - 68 °C

Beispiel 8

5-Phenoxy-4-methoxymethyl-β-carbolin-3-carbonsäureisopropylester

2 g 5-(4-Chlorphenoxy)-4-methoxymethyl-β-carbolin-3-carbonsäureisopropylester werden in 40 ml Isopropanol mit 200 mg Palladium auf Kohle (10 %) und 0,9 ml Triethylamin bei Raumtemperatur unter Normalwasserstoffdruck hydriert. Nach Abfiltrieren vom Katalysator, Einengen des Filtrates und Digerieren des Rückstandes mit Diisopropylether erhält man 1,4 g 5-Phenoxy-4-methoxymethyl-β-carbolin-3-carbonsäureisopropylester vom Schmelzpunkt 201 - 203 °C.

Beispiel 9

5-Phenoxy-4-methoxymethyl-β-carbolin-3-carbonsäure-t-butylester

300 mg 5-Phenoxy-4-methoxymethyl-β-carbolin-3-carbonsäure werden mit 2 ml Aminalester 3 Stunden auf 120 °C erwärmt, wobei eine Lösung entstanden ist. Nach Verdünnen mit Wasser wird mit Essigester extrahiert. Die organische Phase wird getrocknet, filtriert, eingeengt und über Kieselgel mit Aceton : Hexan = 1 : 1 als Elutionsmittel chromatographiert. Man erhält 130 mg 5-Phenoxy-4-methoxymethyl-β-carbolin-3-carbonsäure-t-butylester von einem Zersetzungspunkt von 150 °C.

In analoger Weise werden hergestellt:
5-(4-Chlorphenoxy)-4-methoxymethyl-β-carbolin-3-carbonsäure-t-butylester
Schmelzpunkt 209 - 210 °C
6-(3-Chlorophenoxy)-4-methoxymethyl-β-carbolin-3-carbonsäure-t-butylester
Schmelzpunkt 159 - 160 °C
6-(2-Cyanophenoxy)-4-methoxymethyl-β-carbolin-3-carbonsäure-t-butylester
Schmelzpunkt 144 °C
6-(2-Cyano-3-chlorophenoxy)-4-methoxymethyl-β-carbolin-3-carbonsäure-t-butylester
Schmelzpunkt 100 °C
6-(2,3-Dichlorophenoxy)-4-methoxymethyl-β-carbolin-3-carbonsäure-t-butylester
Schmelzpunkt 203 - 204 °C
6-(4-Fluorphenoxy)-4-methoxymethyl-β-carbolin-3-carbonsäure-t-butylester

Schmelzpunkt 189 - 191 °C
5-(4-Nitrophenoxy)-4-methoxymethyl-β-carbolin-3-carbonsäure-t-butylester
Schmelzpunkt 221 - 222 °C
6-(4-Chlorphenoxy)-4-methyl-β-carbolin-3-carbonsäure-t-butylester
6-Phenoxy-4-methoxymethyl-β-carbolin-3-carbonsäure-t-butylester

Beispiel 10

5-(4-Chlorphenoxy)-4-methoxymethyl-β-carbolin-3-carbonsäureisopropylamid

382 mg 5-(4-Chlorphenoxy)-4-methoxymethyl-β-carbolin-3-carbonsäure werden in 10 ml Dimethylformamid mit 380 mg Carbonyldiimidazol versetzt. Nach 2 Stunden Rühren bei Raumtemperatur wird 1 ml Isopropylamin zugegeben und über Nacht gerührt. Nach Versetzen mit Wasser wird mit Essigester extrahiert. Die Essigesterphase wird getrocknet, filtriert und eingeengt. Der Rückstand wird über Kieselgel mit Methylenchlorid : Aceton = 1 : 1 als Elutionsmittel chromatographiert. Man erhält 90 mg 5-(4-Chlorphenoxy)-4-methoxymethyl-β-carbolin-3-carbonsäureisopropylamid vom Schmelzpunkt 255 °C.

In analoger Weise werden hergestellt:
5-Phenoxy-4-methoxymethyl-β-carbolin-3-carbonsäuremethylamid
Schmelzpunkt 235 °C
5-(4-Chlorphenoxy)-4-methoxymethyl-β-carbolin-3-carbonsäuremethylamid
Schmelzpunkt 236 °C
5-(4-Nitrophenoxy)-4-methoxymethyl-β-carbolin-3-carbonsäureisopropylamid
6-Phenoxy-4-methoxymethyl-β-carbolin-3-carbonsäureisopropylamid
6-(4-Chlorphenoxy)-4-methoxymethyl-β-carbolin-3-carbonsäureisopropylamid
6-(2-Chlorphenoxy)-4-methoxymethyl-β-carbolin-3-carbonsäureisopropylamid
5-Phenoxy-4-methoxymethyl-β-carbolin-3-carbonsäureisopropylamid

Beispiel 11

5-Phenoxy-4-methoxymethyl-β-carbolin-3-carbonsäure

7,7 g 5-Phenoxy-4-methoxymethyl-β-carbolin-3-carbonsäureethylester werden mit 70 ml 2n Natronlauge solange erwärmt, bis eine klare Lösung entstanden ist (2 Stunden). Danach wird in der Wärme vorsichtig mit 5n-Salzsäure angesäuert und darauf 15 Minuten bei Raumtemperatur gerührt. Nach Absaugen und Trocknen über $P_2O_5$ und KOH bei 80 °C erhält man 7,2 g (100 % d.Th.) 5-Phenoxy-4-methoxymethyl-β-carbolin-3-carbonsäure.

In analoger Weise werden hergestellt:
5-(4-Chlorphenoxy)-4-methoxymethyl-β-carbolin-3-carbonsäure
5-(4-Nitrophenoxy)-4-methoxymethyl-β-carbolin-3-carbonsäure
6-(4-Chlorphenoxy)-4-methyl-β-carbolin-3-carbonsäure
6-Phenoxy-4-methoxymethyl-β-carbolin-3-carbonsäure
5-(4-Nitrophenoxy)-4-methyl-β-carbolin-3-carbonsäure
5-(4-Nitrophenoxy)-β-carbolin-3-carbonsäure
6-Phenoxy-β-carbolin-3-carbonsäure
6-Phenoxy-4-methyl-β-carbolin-3-carbonsäure
6-(4-Nitrophenoxy)-β-carbolin-3-carbonsäure
6-(4-Nitrophenoxy)-4-methyl-β-carbolin-3-carbonsäure
6-(4-Chlorphenoxy)-4-methoxymethyl-β-carbolin-3-carbonsäure
6-(4-Bromphenoxy)-4-methoxymethyl-β-carbolin-3-carbonsäure
6-(4-Nitrophenoxy)-4-methoxymethyl-β-carbolin-3-carbonsäure
6-(2-Nitrophenoxy)-4-methoxymethyl-β-carbolin-3-carbonsäure
6-(2-Methyl-4-nitrophenoxy)-4-methoxymethyl-β-carbolin-3-carbonsäure
6-(4-Morpholinosulfamoylphenoxy)-4-methoxymethyl-β-carbolin-3-carbonsäure
6-(4-methylsulfonylphenoxy)-4-methoxymethyl-β-carbolin-3-carbonsäure
6-(4-Diethylsulfamoylphenoxy)-4-methoxymethyl-β-carbolin-3-carbonsäure
6-(2-Chlorphenoxy)-4-methoxymethyl-β-carbolin-3-carbonsäure
6-(4-Cyanophenoxy)-4-methoxymethyl-β-carbolin-3-carbonsäure
6-(4-Fluorphenoxy)-β-carbolin-3-carbonsäure

6-(2-Chlor-4-nitrophenoxy)-4-methoxymethyl-β-carbolin-3-carbonsäure
6-(2,4-Dichlorphenoxy)-4-methoxymethyl-β-carbolin-3-carbonsäure
6-(3-Chlorophenoxy)-4-methoxymethyl-β-carbolin-3-carbonsäure
6-(2-Cyanophenoxy)4-methoxymethyl-β-carbolin-3-carbonsäure
6-(2-Cyano-3-chlorophenoxy)-4-methoxymethyl-β-carbolin-3-carbonsäure
6-(2,3-Dichlorophenoxy)-4-methoxymethyl-β-carbolin-3-carbonsäure
6-(4-Fluorphenoxy)-4-methoxymethyl-β-carbolin-3-carbonsäure

**Patentansprüche**

**1.** Phenoxy-substituierte β-carbolinderivate der allgemeinen Formel I

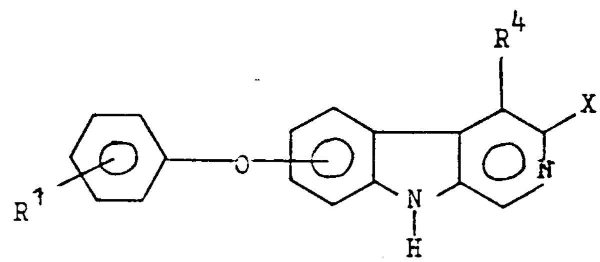

(I),

worin

X einen Oxadiazolylrest der Formel

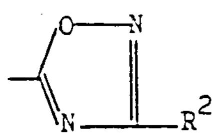

oder

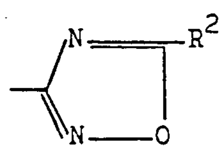

mit $R^2$ in der Bedeutung von H, $C_{1-6}$-Alkyl oder $C_{3-7}$-Cycloalkyl darstellt oder

X eine $COOR^3$-Gruppe mit $R^3$ in der Bedeutung von $C_{1-6}$-Alkyl darstellt oder $CONHC_{1-3}$-Alkyl ist und

$R^4$ Wasserstoff, $C_{1-6}$-Alkyl oder $C_{1-6}$-Alkoxyalkyl bedeutet und

$R^1$ Wasserstoff, Halogen, $C_{1-6}$-Alkyl $C_{1-6}$-Alkoxy, $C_{1-4}$-Alkanoyl, Phenyl, $C_{2-5}$-Alkylendioxy, Trifluormethyl, Cyano, Nitro, $C_{1-6}$-Alkoxycarbonyl, Azido, $SO_2R^6$, $SO_2NR^7R^8$ oder $NR^9R^{10}$ ist,

$R^6$ $C_{1-6}$-Alkyl,

$R^7$, $R^8$ $C_{1-6}$-Alkyl oder gemeinsam mit dem Stickstoffatom einen gesättigten 5- oder 6-gliedrigen Heterocyclus bedeuten, bei dem ein- oder zwei $CH_2$-Gruppen durch Sauerstoff, Schwefel oder Stickstoff ersetzt sein können, und

$R^9$, $R^{10}$ jeweils Wasserstoff, $C_{1-6}$-Alkyl, $C_{1-4}$-Alkanoyl oder gemeinsam mit dem Stickstoffatom einen gesättigten 5- oder 6-gliedrigen Heterocyclus bedeuten, bei dem ein- oder zwei $CH_2$-Gruppen durch Sauerstoff, Schwefel oder Stickstoff ersetzt sein können, und

$R^1$ gleich oder verschieden ein- bis dreifach stehen kann,

wobei X nicht $COOC_2H_5$ ist, wenn

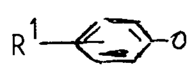 – 5-Phenoxy

und $R^4$ Methyl ist oder wenn

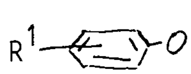 – 6-(4-Methoxyphenoxy)

und $R^4$ Methoxymethyl ist.

**2.** 5-(4-Chlorphenoxy)-3-(3-ethyl-1,2,4-oxadiazol-5-yl)-4-methoxymethyl-β-carbolin
5-Phenoxy-4-methyl-3-(3-ethyl-1,2,4-oxadiazol-5-yl)-β-carbolin
5-(4-Nitrophenoxy)-3-(3-ethyl-1,2,4-oxadiazol-5-yl)-β-carbolin
5-(4-Chlorphenoxy)-4-methyl-3-(3-ethyl-1,2,4-oxadiazol-5-yl)-β-carbolin
6-Phenoxy-4-methyl-3-(3-ethyl-1,2,4-oxadiazol-5-yl)-β-carbolin
6-(4-Chlorphenoxy)-4-methyl-3-(3-ethyl-1,2,4-oxadiazol-5-yl)-β-carbolin
6-(4-Aminophenoxy)-4-methyl-3-(3-ethyl-1,2,4-oxadiazol-5-yl)-β-carbolin
6-(4-Chlorphenoxy)-4-methoxymethyl-3-(3-ethyl-1,2,4-oxadiazol-5-yl)-β-carbolin
6-(4-Chlorphenoxy)-4-methoxymethyl-3-(3-cyclopropyl-1,2,4-oxadiazol-5-yl)-β-carbolin
6-(4-Morpholinosulfamoylphenoxy)-4-methoxymethyl-3-(3-cyclopropyl-1,2,4-oxadiazol-5-yl)-β-carbolin
6-(4-Methylsulfonylphenoxy)-4-methoxymethyl-3-(3-ethyl-1,2,4-oxadiazol-5-yl)-β-carbolin
6-(2-Chlorphenoxy)-4-methoxymethyl-3-(3-ethyl-1,2,4-oxadiazol-5-yl)-β-carbolin
6-(2,4-Dichlorphenoxy)-4-methoxymethyl-3-(3-ethyl-1,2,4-oxadiazol-5-yl)-β-carbolin
5-(4-Nitrophenoxy)-4-methoxymethyl-β-carbolin-3-carbonsäureethylester
5-(4-Nitro-2-chlorphenoxy)-4-methoxymethyl-β-carbolin-3-carbonsäureethylester
6-(4-Nitrophenoxy)-4-methyl-β-carbolin-3-carbonsäureethylester
6-(4-Nitrophenoxy)-4-methoxymethyl-β-carbolin-3-carbonsäureethylester
5-(4-Aminophenoxy)-4-methoxymethyl-β-carbolin-3-carbonsäureethylester
5-Phenoxy-4-methoxymethyl-β-carbolin-3-carbonsäureethylester
5-(3-Chlorphenoxy)-4-methoxymethyl-β-carbolin-3-carbonsäureethylester
5-(2-Chlorphenoxy)-4-methoxymethyl-β-carbolin-3-carbonsäureethylester
6-Phenoxy-4-methoxymethyl-β-carbolin-3-carbonsäureethylester
5-(4-Chlorphenoxy)-4-methoxymethyl-β-carbolin-3-carbonsäureethylester
5-(4-Chlorphenoxy)-4-methyl-β-carbolin-3-carbonsäureethylester
5-(2,4-Dichlorphenoxy)-4-methoxymethyl-β-carbolin-3-carbonsäureethylester
6-(4-Chlorphenoxy)-4-methoxymethyl-β-carbolin-3-carbonsäureethylester
5-(2-Chlorphenoxy)-4-methoxymethyl-3-(3-ethyl-1,2,4-oxadiazol-5-yl)-β-carbolin
5-(3-Chlorphenoxy)-4-methoxymethyl-3-(3-ethyl-1,2,4-oxadiazol-5-yl)-β-carbolin
5-(2-Chlorphenoxy)-4-methoxymethyl-β-carbolin-3-carbonsäureisopropylester
6-(2-Nitrophenoxy)-4-methoxymethyl-β-carbolin-3-carbonsäureisopropylester
6-(2-Cyano-3-chlorphenoxy)-4-methoxymethyl-β-carbolin-3-carbonsäureisopropylester
6-(2-Cyanophenoxy)-4-methoxymethyl-β-carbolin-3-carbonsäureisopropylester
6-(4-Acetylphenoxy)-4-methoxymethyl-3-(3-ethyl-1,2,4-oxadiazol-5-yl)-β-carbolin
6-(3-Methylphenoxy)-4-methoxymethyl-β-carbolin-3-carbonsäureisopropylester
6-(3-Chlorphenoxy)-4-methoxymethyl-β-carbolin-3-carbonsäureisopropylester
6-(2,4-Dichlorphenoxy)-4-methoxymethyl-β-carbolin-3-carbonsäureethylester
5-Phenoxy-4-methoxymethyl-β-carbolin-3-carbonsäureisopropylester
5-(4-Chlorphenoxy)-4-methyl-β-carbolin-3-carbonsäureisopropylester
5-(4-Chlorphenoxy)-4-methoxymethyl-β-carbolin-3-carbonsäureisopropylester
6-(4-Chlorphenoxy)-4-methoxymethyl-β-carbolin-3-carbonsäureisopropylester
6-(4-Fluorphenoxy)-4-methoxymethyl-β-carbolin-3-carbonsäureisopropylester
6-(3,4-Dichlorphenoxy)-4-methoxymethyl-β-carbolin-3-carbonsäureisopropylester
5-Phenoxy-4-methoxymethyl-β-carbolin-3-carbonsäure-t-butylester
6-Phenoxy-4-methoxymethyl-β-carbolin-3-carbonsäure-t-butylester
5-(4-Chlorphenoxy)-4-methoxymethyl-β-carbolin-3-carbonsäureisopropylamid nach Anspruch 1.

**3.** Verfahren zur Herstellung der Verbindungen der allgemeinen Formel I gemäß Anspruch 1 dadurch gekennzeichnet, daß man

a) eine Verbindung der allgemeinen Formel II

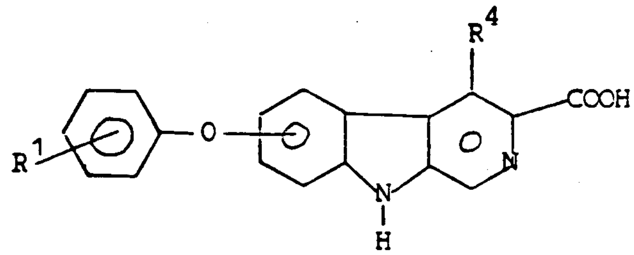

(II)

worin $R^1$ und $R^4$ die oben genannte Bedeutung haben mit einer Verbindung der Formel

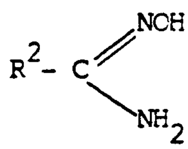

,

worin $R^2$ die oben genannte Bedeutung hat, umsetzt zu einer Verbindung der allgemeinen Formel I, in der X für den Rest

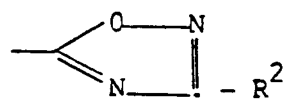

mit $R^2$ in der oben genannten Bedeutung steht,
b) eine Verbindung der allgemeinen Formel III

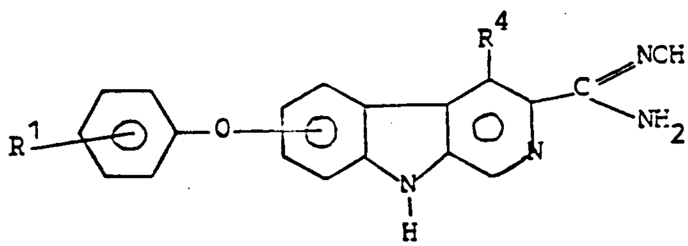

(III),

worin
$R^1$ und $R^4$ die oben genannte Bedeutung haben,
mit einem Carbonsäureanhydrid $(R^2CO)_2O$, worin $R^2$ die oben genannte Bedeutung hat, umsetzt zu einer Verbindung der allgemeinen Formel I, in der X für den Rest

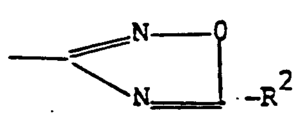

mit $R^2$ in der oben genannten Bedeutung steht,

c) eine Verbindung der allgemeinen Formel IV

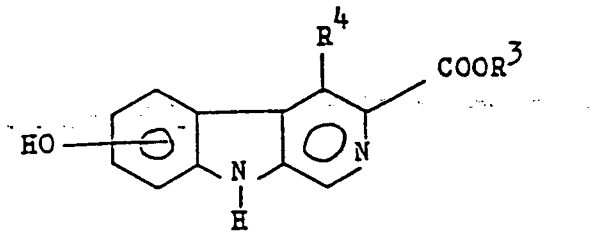

(IV),

worin $R^3$ und $R^4$ die oben genannte Bedeutung haben, mit einer Verbindung der Formel

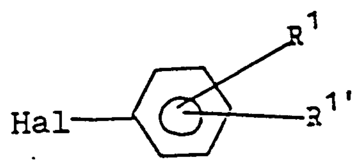

worin Hal Halogen ist

und $R^1$ die oben angegebene Bedeutung hat und $R^{1'}$ einen elektronenziehenden Substituenten darstellt, umsetzt und gegebenenfalls anschließend

α) eine Nitrogruppe zur Aminogruppe reduziert und gewünschtenfalls die so erhaltene Aminogruppe desaminiert oder gegen Ealogen oder Azid austauscht oder

β) falls $R^1$ Halogen ist, katalytisch dehalogeniert oder

γ) eine Estergruppe umestert oder verseift und gewünschtenfalls die so erhaltene Carbonsäure amidiert.

**4.** Verwendung der Verbindungen gemäß Anspruch 1 und 2 zur Herstellung von Arzneimitteln.

**5.** Arzneimittel auf Basis der Verbindungen nach Anspruch 1 und 2.

**6.** Verfahren zur Herstellung eines Arzneimittels dadurch, daß man eine nach Anspruch 3 hergestellte Verbindung der Formel I mit einem pharmazeutischen Träger mischt.

## Claims

**1.** Phenoxy-substituted β-carboline derivatives of the general formula I

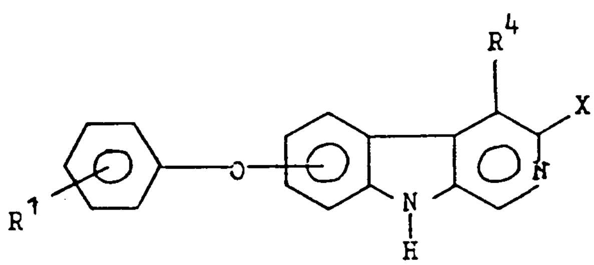

(I)

wherein

X is an oxadiazolyl radical of the formula

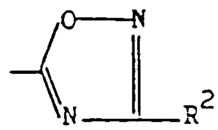
or
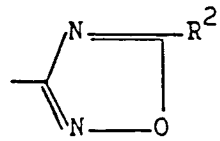

| | |
|---|---|
| | wherein $R^2$ is H, $C_{1-6}$alkyl or $C_{3-7}$cycloalkyl, or |
| X | is a $COOR^3$ group wherein $R^3$ is $C_{1-6}$alkyl or is $CONHC_{1-3}$alkyl, and |
| $R^4$ | is hydrogen, $C_{1-6}$alkyl or $C_{1-6}$alkoxyalkyl and |
| $R^1$ | is hydrogen, halogen, $C_{1-6}$alkyl, $C_{1-6}$alkoxy, $C_{1-6}$alkanoyl, phenyl, $C_{2-5}$alkylenedioxy, trifluoromethyl, cyano, nitro, $C_{1-6}$alkoxycarbonyl, azido, $SO_2R^6$, $SO_2NR^7R^8$ or $NR^9R^{10}$, |
| $R^6$ | is $C_{1-6}$alkyl, |
| $R^7$ and $R^8$ | are $C_{1-6}$alkyl or together with the nitrogen atom form a saturated 5- or 6-membered heterocycle in which one or two $CH_2$ groups may have been replaced by oxygen, sulphur or nitrogen, and |
| $R^9$ and $R^{10}$ | are each hydrogen, $C_{1-6}$alkyl, $C_{1-4}$alkanoyl or together with the nitrogen atom form a saturated 5-or 6-membered heterocycle in which one or two $CH_2$ groups may have been replaced by oxygen, sulphur or nitrogen, and |

there being from 1 to 3 substituents $R^1$, which may be identical or different,
and wherein X is not $COOC_2H_5$ when

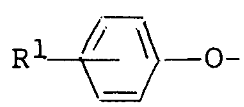

is 5-phenoxy and $R^4$ is methyl or when

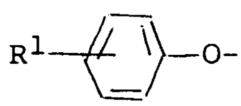

is 6-(4-methoxyphenoxy) and $R^4$ is methoxymethyl.

**2.** 5-(4-Chlorophenoxy)-3-(3-ethyl-1,2,4-oxadiazol-5-yl)-4-methoxymethyl-β-carboline,
5-phenoxy-4-methyl-3-(3-ethyl-1,2,4-oxadiazol-5-yl)-β-carboline,
5-(4-nitrophenoxy)-3-(3-ethyl-1,2,4-oxadiazol-5-yl)-β-carboline,
5-(4-chlorophenoxy)-4-methyl-3-(3-ethyl-1,2,4-oxadiazol-5-yl)-β-carboline,
6-phenoxy-4-methyl-3-(3-ethyl-1,2,4-oxadiazol-5-yl)-β-carboline,
6-(4-chlorophenoxy)-4-methyl-3-(3-ethyl-1,2,4-oxadiazol-5-yl)-β-carboline,
6-(4-aminophenoxy)-4-methyl-3-(3-ethyl-1,2,4-oxadiazol-5-yl)-β-carboline,
6-(4-chlorophenoxy)-4-methoxymethyl-3-(3-ethyl-1,2,4-oxadiazol-5-yl)-β-carboline,
6-(4-chlorophenoxy)-4-methoxymethyl-3-(3-cyclopropyl-1,2,4-oxadiazol-5-yl)-β-carboline,
6-(4-morpholinosulphamoylphenoxy)-4-methoxymethyl-3-(3-cyclopropyl-1,2,4-oxadiazol-5-yl)-β-carboline,
6-(4-methylsulphonylphenoxy)-4-methoxymethyl-3-(3-ethyl-1,2,4-oxadiazol-5-yl)-β-carboline,
6-(2-chlorophenoxy)-4-methoxymethyl-3-(3-ethyl-1,2,4-oxadiazol-5-yl)-β-carboline,
6-(2,4-dichlorophenoxy)-4-methoxymethyl-3-(3-ethyl-1,2,4-oxadiazol-5-yl)-β-carboline,
5-(4-nitrophenoxy)-4-methoxymethyl-β-carboline-3-carboxylic acid ethyl ester,
5-(4-nitro-2-chlorophenoxy)-4-methoxymethyl-β-carboline-3-carboxylic acid ethyl ester,
6-(4-nitrophenoxy)-4-methyl-β-carboline-3-carboxylic acid ethyl ester,
6-(4-nitrophenoxy)-4-methoxymethyl-β-carboline-3-carboxylic acid ethyl ester,
5-(4-aminophenoxy)-4-methoxymethyl-β-carboline-3-carboxylic acid ethyl ester,
5-phenoxy-4-methoxymethyl-β-carboline-3-carboxylic acid ethyl ester,
5-(3-chlorophenoxy)-4-methoxymethyl-β-carboline-3-carboxylic acid ethyl ester,

5-(2-chlorophenoxy)-4-methoxymethyl-β-carboline-3-carboxylic acid ethyl ester,
6-phenoxy-4-methoxymethyl-β-carboline-3-carboxylic acid ethyl ester,
5-(4-chlorophenoxy)-4-methoxymethyl-β-carboline-3-carboxylic acid ethyl ester,
5-(4-chlorophenoxy)-4-methyl-β-carboline-3-carboxylic acid ethyl ester,
5-(2,4-dichlorophenoxy)-4-methoxymethyl-β-carboline-3-carboxylic acid ethyl ester,
6-(4-chlorophenoxy)-4-methoxymethyl-β-carboline-3-carboxylic acid ethyl ester,
5-(2-chlorophenoxy)-4-methoxymethyl-3-(3-ethyl-1,2,4-oxadiazol-5-yl)-β-carboline,
5-(3-chlorophenoxy)-4-methoxymethyl-3-(3-ethyl-1,2,4-oxadiazol-5-yl)-β-carboline,
5-(2-chlorophenoxy)-4-methoxymethyl-β-carboline-3-carboxylic acid isopropyl ester,
6-(2-nitrophenoxy)-4-methoxymethyl-β-carboline-3-carboxylic acid isopropyl ester,
6-(2-cyano-3-chlorophenoxy)-4-methoxymethyl-β-carboline-3-carboxylic acid isopropyl ester,
6-(2-cyanophenoxy)-4-methoxymethyl-β-carboline-3-carboxylic acid isopropyl ester,
6-(4-acetylphenoxy)-4-methoxymethyl-3-(3-ethyl-1,2,4-oxadiazol-5-yl)-β-carboline,
6-(3-methylphenoxy)-4-methoxymethyl-β-carboline-3-carboxylic acid isopropyl ester,
6-(3-chlorophenoxy)-4-methoxymethyl-β-carboline-3-carboxylic acid isopropyl ester,
6-(2,4-dichlorophenoxy)-4-methoxymethyl-β-carboline-3-carboxylic acid ethyl ester,
5-phenoxy-4-methoxymethyl-β-carboline-3-carboxylic acid isopropyl ester,
5-(4-chlorophenoxy)-4-methyl-β-carboline-3-carboxylic acid isopropyl ester,
5-(4-chlorophenoxy)-4-methoxymethyl-β-carboline-3-carboxylic acid isopropyl ester,
6-(4-chlorophenoxy)-4-methoxymethyl-β-carboline-3-carboxylic acid isopropyl ester,
6-(4-fluorophenoxy)-4-methoxymethyl-β-carboline-3-carboxylic acid isopropyl ester,
6-(3,4-dichlorophenoxy)-4-methoxymethyl-β-carboline-3-carboxylic acid isopropyl ester,
5-phenoxy-4-methoxymethyl-β-carboline-3-carboxylic acid tert.-butyl ester,
6-phenoxy-4-methoxymethyl-β-carboline-3-carboxylic acid tert.-butyl ester, or
5-(4-chlorophenoxy)-4-methoxymethyl-β-carboline-3-carboxylic acid isopropylamide, according to claim 1.

**3.** A process for the preparation of the compounds of the general formula I according to claim 1, characterised in that

a) a compound of the general formula II

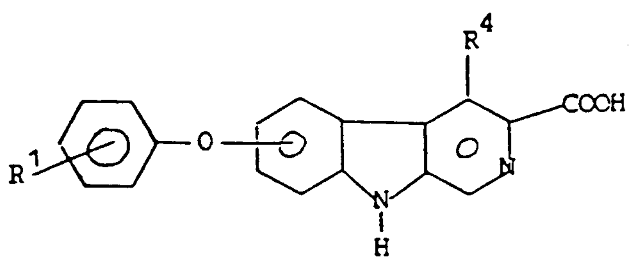

(II)

wherein $R^1$ and $R^4$ have the meanings given above, is reacted with a compound of the formula

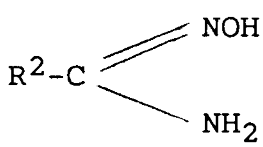

,

wherein $R^2$ has the meaning given above, to form a compound of the general formula I wherein X is the radical

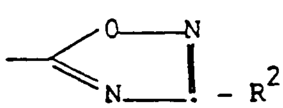

wherein $R^2$ has the meaning given above,
b) a compound of the general formula III

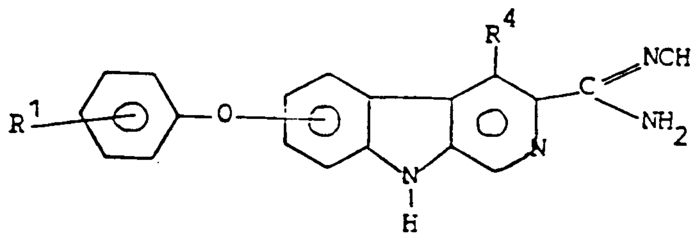

(III),

wherein
$R^1$ and $R^4$ have the meanings given above, is reacted with a carboxylic acid anhydride $(R^2CO)_2O$, wherein $R^2$ has the meaning given above, to form a compound of the general formula I wherein X is the radical

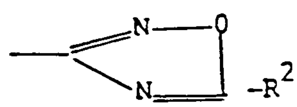

wherein $R^2$ has the meaning given above,
c) a compound of the general formula IV

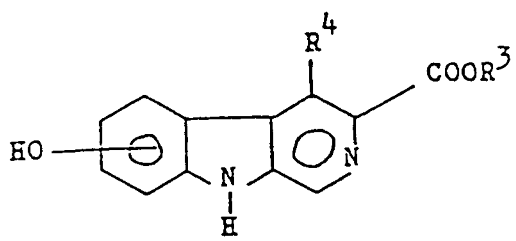

(IV)

wherein $R^3$ and $R^4$ have the meanings given above, is reacted with a compound of the formula

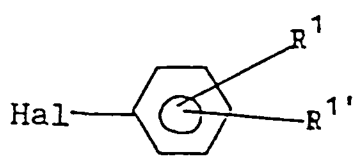

wherein Hal is halogen and
$R^1$ has the meaning given above and $R^{1'}$ is an electrophilic substituent, and optionally then
α) a nitro group is reduced to the amino group and, if desired, the resulting amino group is deaminated or replaced by halogen or azide, or
β) when $R^1$ is halogen, is catalytically dehalogenated, or
γ) an ester group is transesterified or hydrolysed and, if desired, the resulting carboxylic acid is amidated.

**4.** The use of the compounds according to claims 1 and 2 for the preparation of medicaments.

**5.** Medicaments based on the compounds according to claims 1 and 2.

**6.** A process for the preparation of a medicament by mixing a compound of the formula I prepared in accordance with claim 3 with a pharmaceutical carrier.

## Revendications

1. Dérivés phénoxy-substitués de la $\beta$-carboline de formule générale I

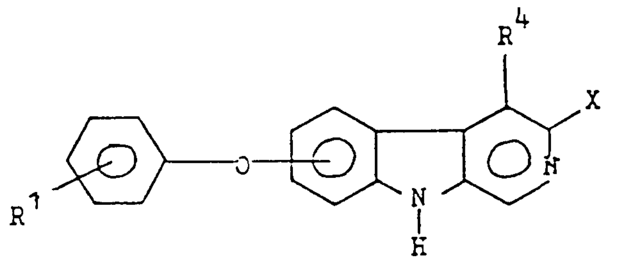

(I)

où

X est un radical oxadiazolyle de formule

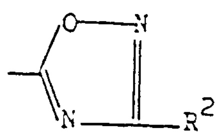

ou

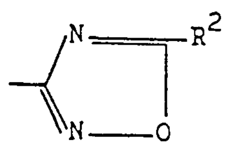

$R^2$ représentant H ou un radical alkyle en $C_{1-6}$ ou cycloalkyle en $C_{3-7}$, ou bien

X représente un groupe $COOR^3$, $R^3$ étant un radical alkyle en $C_{1-6}$, ou encore CONH-(alkyle en $C_1$-$C_3$), et

$R^4$ est un hydrogène ou un radical alkyle en $C_1$-$C_6$ ou alcoxyalkyle en $C_1$-$C_6$, et

$R^1$ est un hydrogène ou un radical halogéno, alkyle en $C_{1-6}$, alcoxy en $C_{1-6}$, alcanoyle en $C_{1-4}$, phényle, alkylènedioxy en $C_{2-5}$, trifluorométhyle, cyano, nitro, (alcoxy en $C_1$-$C_6$)-carbonyle, azido, $SO_2R^6$, $SO_2NR^7R^8$ ou $NR^9R^{10}$,

$R^6$ est un radical alkyle en $C_{1-6}$,

$R^7$ et $R^8$ représentent chacun un radical alkyle en $C_{1-6}$, ou encore représentent ensemble avec l'atome d'azote un hétérocycle saturé à cinq ou six chaînons, dont un ou deux groupes $CH_2$ peuvent être remplacés par des atomes d'oxygène, de soufre ou d'azote, et

$R^9$ et $R^{10}$ représentent chacun un hydrogène ou un radical alkyle en $C_{1-6}$, alcanoyle en $C_{1-4}$, ou forment ensemble avec l'atome d'azote un hétérocycle saturé à cinq ou six chaînons dont un ou deux groupes $CH_2$ peuvent être remplacés par des atomes d'oxygène, de soufre ou d'azote, et

$R^1$ peut être présent une à trois fois, en représentant des radicaux identiques ou différents,

du moment que X n'est pas $COOC_2H_5$ quand

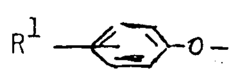

est un radical phénoxy en 5 et $R^4$ est le radical méthyle, ou encore quand

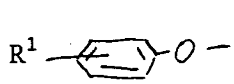

est un radical 4-méthoxyphénoxy en 6 et $R^4$ le radical méthoxyméthyle.

2. 5-(4-chlorophénoxy)-3-(3-éthyl-1,2,4-oxadiazole-5-yl)-4-méthoxyméthyl-$\beta$-carboline
   5-phénoxy-4-méthyl-3-(3-éthyl-1,2,4-oxadiazole-5-yl)-$\beta$-carboline
   5-(4-nitrophénoxy)-3-(3-éthyl-1,2,4-oxadiazole-5-yl)-$\beta$-carboline
   5-(4-chlorophénoxy)-4-méthyl-3-(3-éthyl-1,2,4-oxadiazole-5-yl)-$\beta$-carboline
   6-phénoxy-4-méthyl-3-(3-éthyl-1,2,4-oxadiazole-5-yl)-$\beta$-carboline
   6-(4-chlorophénoxy)-4-méthyl-3-(3-éthyl-1,2,4-oxadiazole-5-yl)-$\beta$-carboline
   6-(4-aminophénoxy)-4-méthyl-3-(3-éthyl-1,2,4-oxadiazole-5-yl)-$\beta$-carboline

6-(4-chlorophénoxy)-4-méthoxyméthyl-3-(3-éthyl-1,2,4-oxadiazole-5-yl)-β-carboline
6-(4-chlorophénoxy)-4-méthoxyméthyl-3-(3-cyclopropyl-1,2,4-oxadiazole-5-yl)-β-carboline
6-(4-morpholinosulfamoylphénoxy)-4-méthoxyméthyl-3-(3-éthyl-1,2,4-oxadiazole-5-yl)-β-carboline
6-(4-méthylsulfonylphénoxy)-4-méthoxyméthyl-3-(3-éthyl-1,2,4-oxadiazole-5-yl)-β-carboline
6-(2-chlorophénoxy)-4-méthoxyméthyl-3-(3-éthyl-1,2,4-oxadiazole-5-yl)-β-carboline
6-(2,4-dichlorophénoxy)-4-méthoxyméthyl-3-(3-éthyl-1,2,4-oxadiazole-5-yl)-β-carboline
ester éthylique de l'acide 5-(4-nitrophénoxy)-4-méthoxyméthyl-β-carboline-3-carboxylique
ester éthylique de l'acide 5-(4-nitro-2-chlorophénoxy)-4-méthoxméthyl-β-carboline-3-carboxylique
ester éthylique de l'acide 6-(4-nitrophénoxy)-4-méthyl-β-carboline-3-carboxylique
ester éthylique de l'acide 6-(4-nitrophénoxy)-4-méthoxyméthyl-β-carboline-3-carboxylique
ester éthylique de l'acide 5-(4-aminophénoxy)-4-méthoxyméthyl-β-carboline-3-carboxylique
ester éthylique de l'acide 5-phénoxy-4-méthoxyméthyl-β-carboline-3-carboxylique
ester éthylique de l'acide 5-(3-chlorophénoxy)-4-méthoxyméthyl-β-carboline-3-carboxylique
ester éthylique de l'acide 5-(2-chlorophénoxy)-4-méthoxyméthyl-β-carboline-3-carboxylique
ester éthylique de l'acide 6-phénoxy-4-methoxyméthyl-β-carboline-3-carboxylique
ester éthylique de l'acide 5-(4-chlorophénoxy)-4-méthoxyméthyl-β-carboline-3-carboxylique
ester éthylique de l'acide 5-(4-chlorophénoxy)-4-méthyl-β-carboline-3-carboxylique
ester éthylique de l'acide 5-(2,4-dichlorophénoxy)-4-méthoxyméthyl-β-carboline-3-carboxylique
ester éthylique de l'acide 6-(4-chlorophénoxy)-4-méthoxyméthyl-β-carboline-3-carboxylique
5-(2-chlorophénoxy)-4-méthoxyméthyl-3-(3-éthyl-1,2,4-oxadiazol-5-yl)-β-carboline
5-(3-chlorophénoxy)-4-méthoxyméthyl-3-(3-éthyl-1,2,4-oxadiazol-5-yl)-β-carboline
ester isopropylique de l'acide 5-(2-chlorophénoxy)-4-méthoxyméthyl-β-carboline-3-carboxylique
ester isopropylique de l'acide 6-(2-chlorophénoxy)-4-méthoxyméthyl-β-carboline-3-carboxylique
ester isopropylique de l'acide 6-(2-cyano-3-chlorophénoxy)-4-méthoxyméthyl-β-carboline-3-carboxylique
ester isopropylique de l'acide 6-(2-cyanophénoxy)-4-méthoxyméthyl-β-carboline-3-carboxylique
6-(4-acétylphénoxy)-4-méthoxméthyl-3-(3-éthyl-1,2,4-oxadiazol-5-yl)-β-carboline
ester isopropylique de l'acide 6-(3-méthylphénoxy)-4-méthoxyméthyl-β-carboline-3-carboxylique
ester isopropylique de l'acide 6-(3-chlorophénoxy)-4-méthoxyméthyl-β-carboline-3-carboxylique
ester éthylique de l'acide 6-(2,4-dichlorophénoxy)-4-méthoxyméthyl-β-carboline-3-carboxylique
ester isopropylique de l'acide 5-phénoxy-4-méthoxyméthyl-β-carboline-3-carboxylique
ester isopropylique de l'acide 5-(4-chlorophénoxy)-4-méthyl-β-carboline-3-carboxylique
ester isopropylique de l'acide 5-(4-chlorophénoxy)-4-méthoxyméthyl-β-carboline-3-carboxylique
ester isopropylique de l'acide 6-(4-chlorophénoxy)-4-méthoxyméthyl-β-carboline-3-carboxylique
ester isopropylique de l'acide 6-(4-fluorophénoxy)-4-méthoxyméthyl-β-carboline-3-carboxylique
ester isopropylique de l'acide 6-(3,4-dichlorophénoxy)-4-méthoxyméthyl-β-carboline-3-carboxylique
ester tert-butylique de l'acide 5-phénoxy-4-méthoxyméthyl-β-carboline-3-carboxylique
ester tert-butylique de l'acide 6-phénoxy-4-méthoxyméthyl-β-carboline-3-carboxylique
isopropylamide de l'acide 5-(4-chlorophénoxy)-4-méthoxyméthyl-β-carboline-3-carboxylique, selon la revendication 1.

3. Procédé pour préparer les composés de formule générale I selon la revendication 1, caractérisé en ce que :

a) on fait réagir un composé de formule générale II

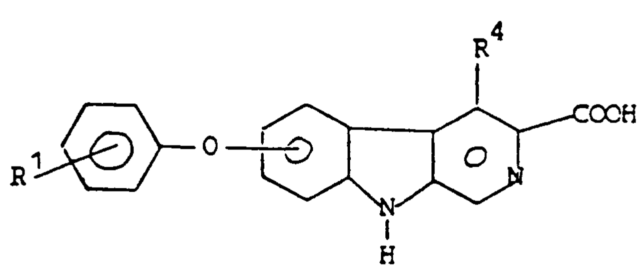

(II)

dans laquelle $R^1$ et $R^4$ ont les significations données ci-dessus, avec un composé de formule

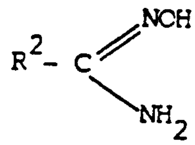

dans laquelle $R^2$ a les significations données ci-dessus, pour obtenir un composé de formule générale I, dans laquelle X représente le radical

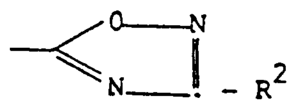

où $R^2$ a les significations données ci-dessus,
b) on fait réagir un composé de formule générale III

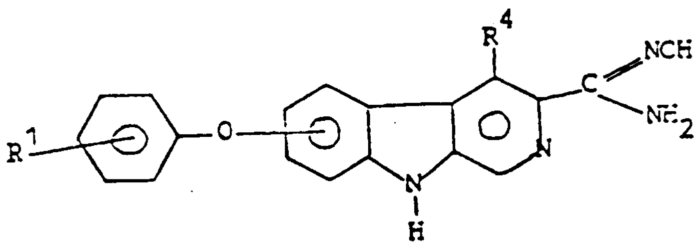

(III)

dans laquelle $R^1$ et $R^4$ ont les significations données ci-dessus, avec un anhydride d'acide carboxylique $(R^2CO)_2O$, où $R^2$ a les significations données ci-dessus, pour obtenir un composé de formule générale I, dans laquelle X représente le radical

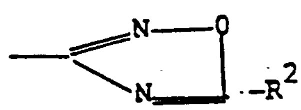

où $R^2$ a les significations données ci-dessus,
c) on fait réagir un composé de formule générale IV

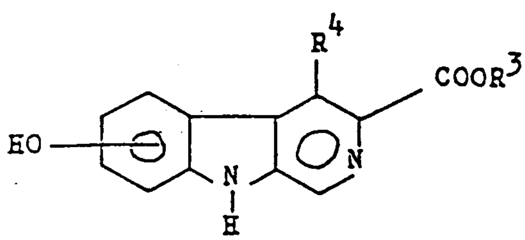

(IV)

dans laquelle $R^3$ et $R^4$ ont les significations données ci-dessus, avec un composé de formule

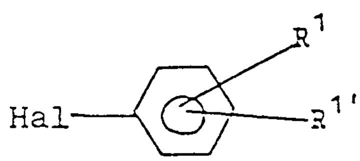

dans laquelle $R^1$ a les significations données ci-dessus, et $R^{1'}$ représente un substituant attracteur d'électrons,
puis, éventuellement,

α) on réduit un groupe nitro en le groupe amino et, si on le souhaite, on désamine le groupe amino ainsi obtenu ou on le remplace par des groupes halogéno ou azido, ou bien
β) si $R^1$ est un halogène, on procède à une déshalogénation catalytique, ou bien
γ) on transestérifie un groupe ester ou on le saponifie, et éventuellement on procède à une amidation de l'acide carboxylique ainsi obtenu.

**4.** Utilisation des composés selon les revendications 1 et 2 pour préparer des médicaments.

**5.** Médicament à base des composés selon les revendications 1 et 2.

**6.** Procédé pour préparer un médicament, caractérisé en ce qu'on mélange un composé de formule I, préparé selon la revendication 3, avec un excipient pharmaceutique.